# FASCICULE DE BREVET EUROPEEN

(11) **EP 1 409 461 B1**
(45) Date de publication et mention de la délivrance du brevet: **23.02.2011**
(21) Numéro de dépôt: 02762543.3
(22) Date de dépôt: 11.07.2002
(51) Int. Cl.: C07D 233/60, C07D 405/06, A61K 31/4164, A61K 31/4178, A61P 31/10

(54) **NOUVEAUX DERIVES D'AZOLE OU DE TRIAZOLE, LEUR PROCEDE DE PREPARATION ET LEUR APPLICATION COMME FONGICIDES**
AZOL- ODER TRIAZOLDERIVATE, VERFAHREN ZU DEREN HERSTELLUNG UND IHRE VERWENDUNG ALS FUNGIZIDE
NOVEL AZOLE OR TRIAZOLE DERIVATIVES, METHOD FOR PREPARING SAME AND USE THEREOF AS FUNGICIDES

(30) Priorité: 13.07.2001 FR 0109331
(43) Date de publication de la demande: 21.04.2004
(73) Titulaire: Aventis Pharma S.A., 92160 Antony (FR)
(72) Inventeur: BABIN, Didier, F-78180 Montigny (FR); WESTON, John, 65779 Kelkheim (DE)
(74) Mandataire: Le Coupanec, Pascale A.M.P.
(86) Numéro de dépôt international: PCT/FR2002/002435
(87) Numéro de publication internationale: WO 2003/006436

(56) Documents cités:
- EP-A- 0 121 753
- WO-A-00/20413
- US-A- 2 601 275

## Description

La présente invention concerne de nouveaux dérivés d'azole ou de triazole, leur procédé de préparation et leur application comme fongicides.

De nombreux composés ayant une activité antifongique sont connus dans l'art antérieur. On peut citer notamment des dérivés d'azoles tels que définis dans les demandes suivantes : EP 0 121 753 A (Hoechst AG), EP 0 050 298 A (Hoechst AG), US 2,813,872 (J schmutz), WO 00/20413 (Hoechst Marion Roussel). Par ailleurs, les nouveaux composés antifongiques doivent pouvoir présenter une solubilité améliorée et doivent pouvoir également être plus facilement absorbés. Il existe néanmoins un réel besoin de mettre en oeuvre de nouveaux composés antifongiques, les souches actuelles pouvant être ou devenir résistantes aux agents conventionnels notamment lorsque ces derniers ne possèdent qu'une activité fongistatique. Enfin, l'incidence du *Candida albicans* comme agent infectieux, est de plus en plus importante, notamment vis-à-vis des patients immunodéprimés, par exemple suite à une infection au VIH, et nécessite donc de nouveaux traitements.

L'objectif de la présente invention est de fournir de nouveaux composés ayant une activité antifongique, notamment vis-à-vis des souches *Candida* ou *Aspergillus.*

L'invention a pour objet, les composés de formule (Ia) ou (Ib) 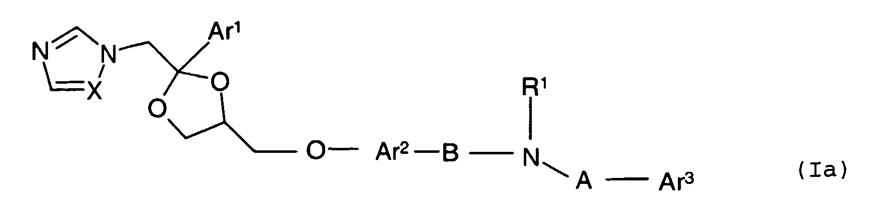 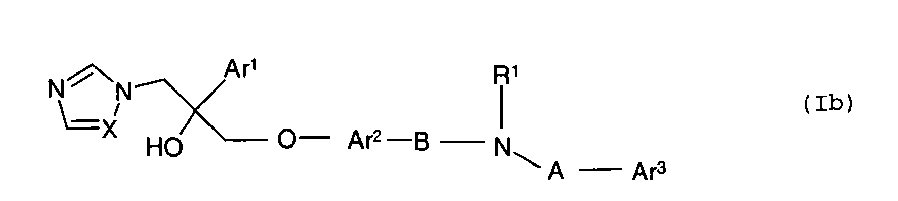 dans lesquelles
- X est un atome d'azote ou un groupement CH,
- Ar¹ représente un aryle carbocyclique ou hétérocyclique non substitué ou substitué par un ou plusieurs radicaux R², R³ ou R⁴
- Ar² représente un phénylène ou naphtylène non substitué ou substitué par un ou plusieurs radicaux R⁵, R⁶ ou R⁷
- Ar³ représente un aryle carbocyclique ou hétérocyclique non substitué ou substitué par un ou plusieurs radicaux R⁸, R⁹ ou R¹⁰
- A représente un radical (C₁-C₄)-alkylène,
- B représente un radical -CH=CH-(C₁-C₄)-alkylène- ou un radical -cyclopropylène-(C₁-C₄)-alkylène-, les dits radicaux cyclopropylène ou -CH=CH- étant non substitués ou substitués par un radical R² et/ou R³,
- R¹ représente un atome d'hydrogène, un groupement -SO₃H ou bien un radical (C₁-C₆)-alkyle non substitué ou substitué par un radical tel que défini pour R², ou un radical alkylène lié au group Ar³
- R², R³, R⁴, R⁵, R⁶, R⁷, R⁸, R⁹ ou R¹⁰ identiques ou différents, représentent fluor, chlore, brome, cyano, mono- bi- ou trihalogeno(C₁-C₈)alkyle, mono- bi- ou trihalogeno(C₁-C₈)-alkyloxy, hydroxy, nitro, carboxyle, formyle, -SO₃H, -OSO₃H, (R¹¹O)₂P(O)-, (R¹¹O)₂P(O)-O-, amino, (C₁-C₈)-alkylamino, di((C₁-C₈)alkyl) amino, (C₅-C₁₄)-aryl-(C₁-C₆)-alkylènamino ou (C₅-C₁₄) -arylamino, (C₁-C₈) -alkyle, (C₅-C₁₄)-aryle, un hétérocycle éventuellement substitué par oxo, (C₅-C₁₄)-aryl- (C₁-C₆) alkyle, amino- (C₁-C₆)-alkyle, (C₁-C₈)-alkylamino- (C₁-C₆) -alkyle, di- ((C₁-C₈)alkyl) amino- (C₁-C₆)-alkyle, hydroxy-(C₁-C₆)alkyle, (C₁-C₆)-alkyloxy-(C₁-C₆)-alkyle, (C₁-C₈)-alkyloxy éventuellement interrompu par un ou plusieurs atomes d'oxygène, (C₅-C₁₄)-aryl-(C₁-C₆)-alkylènoxy, (C-C₁₄)-aryloxy, hydroxy-(C₁-C₆) alkylènoxy, (C₁-C₆)-alkyloxy- (C₁-C₆) alkylènoxy, amino- (C₁-C₆)-alkylènoxy, (C₁-C6) -alkylamino- (C₁-C₆)-alkylènoxy, di((C₁-C₈)-alkyl)amino-(C₁-C₆)-alkylènoxy, méthylènedioxy, (C₁-C₆)-alkyloxycarbonyle, (C₁-C₆)-alkylcarbonyle, (C₅-C₁₄)aryl-(C₁-C₆)-alkylènecarbonyl, (C₅-C₁₄)-aryl-carbonyle, (C₁-C₆)-alkylaminocarbonyle, (C₁-C₆)alkanoylamino, (C₁-C₆)-alkyl= sulfonylamino, (C₅-C₁₄)-arylsulfonylamino, (C₅-C₁₄)-aryl-(C₁-C₆)-alkylènesulfonylamino, (C₁-C₆)-alkylaminosulfonyle, (C₅-C₁₄) -aryl- (C₁-C₆)-alkylènaminosulfonyl, (C₁-C₆)-alkyl-sulfonyl, (C₅-C₁₄)-aryl-(C₁-C₈)-alkylènesulfonyle ou (C₅-C₁₄)-aryl-sulfonyle, les dits radicaux alkyles, aryles ou hétérocycles étant eux même non substitués ou substitués par un ou plusieurs groupements cités ci-dessus.
- R¹¹ représente hydrogène, (C₁-C₁₀)-alkyl, (C₆-C₁₄)-aryle ou (C₆-C₁₄) -aryl- (C₁-C₆)-alkyle,
sous toutes leurs formes stéréoisomères possibles et leurs mélanges,ainsi que leurs sels d'addition physiologiquement acceptables et leurs prodrogues.

Tous les radicaux qui peuvent se retrouver plusieurs fois dans les composés de formule (I) (on entend par (I) : (Ia) ou (Ib)), par exemple, le radical R², sont indépendants les uns des autres et peuvent être identiques ou différents.

Les radicaux alkyles cités plus haut peuvent être linéaires, ramifiés ou cyclique, saturés ou mono- ou poly-insaturés. Ceci s'applique également lorsqu'ils portent un substituant ou lorsqu'ils sont inclus dans des groupements tels que par exemple alkoxy, alkoxycarbonyle ou aralkyle.

Par (C₁-C₈)-alkyle saturé on entend les radicaux méthyle, éthyle, propyle, butyle, pentyle, hexyle, heptyle, octyle, nonyle, les n-isomères de ces radicaux, isopropyle, isobutyle, isopentyle, néopentyle, isohexyle, 3-méthylepentyle, 2,3,4-triméthylhexyle, sec-butyle, tert-butyle, tert-pentyle. Parmi les radicaux préférés on peut citer méthyle, éthyle, n-propyle, isopropyle, n-butyle, isobutyle, et tert-butyle. Par (C₁-C₆)-alkyle on entend les radicaux méthyle, éthyle, propyle, butyle, pentyle, hexyle et les n-isomères de ces radicaux.

Par radical alkyloxy interrompu par un ou plusieurs atomes d'oxygènes, on entend de préférence des radicaux du type O-CH₂-O-(CH₂)₂-O-CH₃.

Les radicaux alkylènes bivalents correspondant aux radicaux monovalents cités plus haut sont par exemple les radicaux méthylène, éthylène, 1,3-propylène, 1,2-propylène (=1-méthyléthylène), 2,3-butylène (=1,2-diméthyléthylène), 1,4-butylène, ou 1,6-hexylène.

Les radicaux alkyles insaturés peuvent contenir une ou plusieurs, par exemple une, deux ou trois double et/ou triple liaison. Bien entendu, un radical alkyle insaturé contient au moins deux atomes de carbone. Par radical alkyle insaturé on entend donc par exemple, les radicaux alkényles tels que vinyle, 1-propényle, allyle, butényle, 3-méthyle-2-butényle ou les radicaux alkynyles tels que éthynyle, 1-propynyle ou propargyle.

Par radicaux alkylène bivalents insaturés on entend les radicaux alkénylène et alkynylène qui peuvent également être linéaires ou ramifiés. Il s'agit par exemple des radicaux vinylène, propénylène, éthynylène ou propynylène.

Les radicaux cycloalkyles peuvent être monocycliques, bicycliques ou tricycliques. Il s'agit par exemple des radicaux cyclopropyle, cyclobutyle, cyclopentyle, cyclohexyle, cycloheptyle, cyclooctyle, cyclononyle, cyclodécyle, cycloundécyle, cyclododécyle, cyclotétradécyle ou cyclooctadécyle qui le cas échéant peuvent être substitués par exemple par un alkyle renfermant de 1 à 4 atomes de carbone. Comme radicaux cycloalkyles substitués, on peut citer le 4-méthylcyclohexyle, le 2,3-diméthylcyclohexyle, le diméthylcyclopropane et le dichlorocyclopropane.

Sauf indication contraire, les radicaux alkyles ou cycloalkyles peuvent être non substitués ou substitués par un ou plusieurs radicaux identiques ou différents choisis parmi (C₁-C₆)-alkyle, (C₁-C₆)-alkoxy, hydroxyle, halogène tel que fluor, chlore et brome, nitro, amino, trifluorométhyle, mono, -OCF₃, cyano, carboxyl, -SO₃H, -OSO₃H, PO₃H₂, OPO₃H₂, (C₁-C₄)-alkoxycarbonyle, phényle, phénoxy, benzyle et benzyloxy. Bien entendu ceci s'applique également lorsque le radical alkyle fait parti d'un groupement le renfermant, par exemple tel que, (C₁-C₆)-alkyloxycarbonyle, (C₁-C₆)-alkylcarbonyle ou (C₁-C₆)-alkylaminocarbonyle

Par halogène on entend fluor, chlore, brome ou iode.

Par le terme aryle on entend :
- soit les radicaux (C₅-C₁₄)-aryle hétérocycliques (= (C₅-C₁₄)-hétéroaryle), dans lesquels les atomes de carbone du cycle sont remplacés par un hétéroatome tel que azote, oxygène ou soufre,
- soit les radicaux (C₆-C₁₄)-aryle carbocyclique.

Parmi les radicaux(C₆-C₁₄)-aryle carbocycliques, on peut citer le phényle, le naphtyle, le biphénylyle, l'anthryle ou le fluorényle et tout particulièrement le 1-naphtyle, le 2-naphtyle et le phényle.

Sauf indication contraire, les radicaux aryles, en particulier phényle, peuvent être non substitués ou substitués par un ou plusieurs radicaux identiques ou différents choisis parmi (C₁-C₆)-alkyle, (C₁-C₆)-alkoxy, hydroxyle, hydroxy(C₁-C₆)-alkyle, halogène tel que fluor, chlore et brome, nitro, amino, trifluorométhyle, -OCF₃, cyano, carboxyl, -SO₃H, -OSO₃H, PO₃H₂, OPO₃H₂, (C₁-C₄)-alkoxycarbonyle, phényle, phénoxy, benzyle, benzyloxy et méthylènedioxy.

Dans le cas du phényle monosubstitué, le substituant peut être situé en position 2, 3 ou 4, et de préférence en position 3 ou 4. De préférence, Ar³ représente un phényle substitué en position 4. Dans le cas où le phényle est disubstitué, les substituants peuvent être en position 2,3 ou 2,4 ou 2,5 ou 2,6 ou 3,4 ou 3,5. De préférence, lorsque Ar¹ représente un phényle disubstitué, les deux substituants sont en position 2,4. Lorsque ce phényle est tri-substitué les positions sont les suivants : 2,3,4 ou 2,3,5 ou 2,3,6 ou 2,4,5 ou 2,4,6 ou 3,4,5. De la même manière, les radicaux naphtyles ou d'autres radicaux aryles peuvent être substitués en toute position, par exemple le radical 1-naphtyle en position 2-, 3-, 4-, 5-, 6-, 7-, et 8 et le radical 2-naphtyle en position 1-, 3-, 4-, 5-, 6-, et 7.

Le groupement (C₅-C₁₄)-aryle peut représenter également un système aromatique monocyclique ou polycyclique dans lequel 1,2,3 ou 4 atomes de carbone du cycle sont remplacés par des hétéroatomes, en particulier identiques ou différents du groupe constitué de l'azote, l'oxygène et le soufre. Parmi les groupements (C₅-C₁₄)-aryle hétérocycliques (= (C₅-C₁₄)-hétéroaryle) on peut citer les groupements 2-pyridyle, 3-pyridyle, 4-pyridyle, pyrrolyle, furyle, thiényle, imidazolyle, pyrazolyle, oxazolyle, isoxazolyle, thiazolyle, isothiazolyle, tétrazolyle, pyridyle, pyrazinyle, pyrimidinyle, indolyle, isoindolyle, indazolyle, phtalazinyle, quinolyle, isoquinolyle, quinoxalinyle, quinazolinyle, cinnolinyle, β-carbolinyle, ou encore des dérivés benzocondensés, cyclopenta-, cyclohexa-, ou cyclohepta- condensés de ces radicaux.

Le système hétérocyclique peut être substitué par les mêmes substituants cités plus haut pour le système carbocyclique.

Bien entendu, le description ci-dessus concernant les groupes aryles s'applique également lorsque aryle est un radical inclus dans un groupement tel que aryl-alkyle. Comme exemples préférés de groupements aryl-alkyle, on peut citer benzyle, 1-phényléthyl ou 2-phényléthyle.

Par hétérocycle, on entend de préférence un radical à 5 ou 6 chaînons, non aromatique, renfermant éventuellement une ou deux doubles liaisons et un ou plusieurs atomes d'azote ou d'oxygène substitué ou non substitué par les mêmes substituants cités plus haut pour le système carbocyclique ainsi que le radical oxo. L'invention comprend ainsi les hétérocycles suivants :

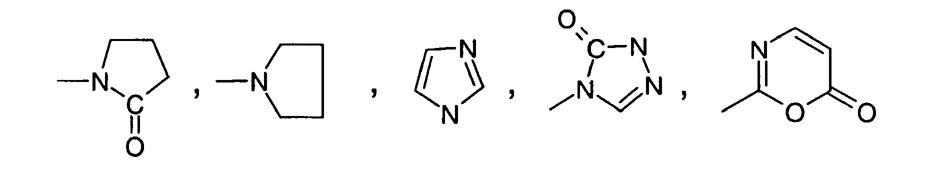

Ces hétérocycles pouvant être substitués. Il peut alors s'agir des radicaux suivants : 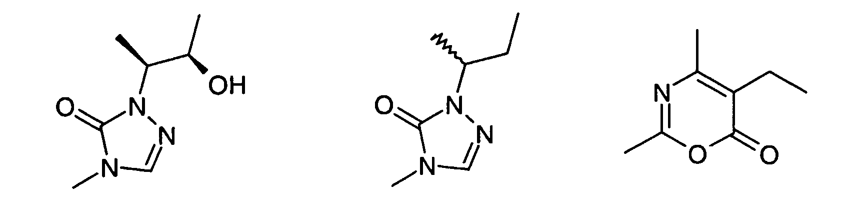 Les atomes de carbone optiquement actifs contenus dans les composés de formule (I) peuvent indépendamment les uns des autres présenter la configuration R ou la configuration S.

Les composés de formule (I) peuvent être sous la forme d'énantiomères purs ou de diastéréoisomères purs ou sous la forme d'un mélange d'énantiomères, par exemple sous forme de racémates ou de mélanges de diastéréoisomères.

La présente invention a donc pour objet les énantiomères purs, les mélanges de ces énantiomères, les diastéréoisomères purs et les mélanges de ces diastéréoisomères.

L'invention comprend les mélanges de deux ou plus de deux stéréoisomères de formule (I) et tous les rapports de ces stéréoisomères au sein des dits mélanges.

Les composés de formule (I) peuvent le cas échéant être présent sous forme d'isomères E ou d'isomères Z. L'invention a donc pour objet les isomères E purs, les isomères Z purs et les mélanges E/Z selon un rapport quelconque. Lorsque les composés de formule (I) renferment un cyclopropane, ces composés de formule (I) peuvent être présent sous forme d'isomères cis ou trans. L'invention a donc pour objet les isomères cis purs, les isomères trans purs et les mélanges cis/trans selon un rapport quelconque.

L'invention comprend également toutes les formes tautomères des composés de formule (I). Les diastéréoisomères, incluant les isomères E/Z (ou cis/trans) peuvent être séparés en isomères individuels, par exemple par chromatographie. Les racémates peuvent être séparés en deux énantiomères par des méthodes courantes telles que la chromatographie en phase chirale ou par des méthodes de résolution.

Les sels physiologiquement acceptables des composés de formule (I) sont en particulier des sels pharmaceutiquement utilisables ou non toxiques ou physiologiquement utilisables.

Lorsque les composés de formule (I) renferment un groupe acide tel que l'acide carboxylique, il s'agit par exemple des sels de métaux alcalins ou alcalino terreux tels que les sels de sodium, de potassium, de magnésium, de calcium, et également les sels formés avec des ions ammonium quaternaires physiologiquement acceptables et les sels d'addition avec les acides tel que l'ammoniac et des amines organiques physiologiquement acceptables telles que par exemple la triéthylamine, l'éthanolamine ou le tris-(2-hydroxyéthyle)amine.

Lorsque les composés de formule (I) contiennent un groupe basique, ils peuvent former un sel d'addition avec les acides par exemple avec les acides inorganiques tels que l'acide chlorhydrique, sulfurique, phosphorique ou avec les acides organiques carboxyliques tels que l'acide acétique, trifluoroacétique, citrique, benzoique, maléique, fumarique, tartarique, méthanesulfonique ou para toluène sulfonique.

Les composés de formule (I) qui comportent un groupe basique et un groupe acide, tel que par exemple guanidino et carboxylique, peuvent être présents sous forme de Zwiterions (bétaînes), qui sont également inclus dans la présente invention.

Lorsque les composés de formule (I) renferment un groupement ammonium chargé, le contre anion (Q⁻) est de préférence un anion monovalent ou un équivalent d'anion polyvalent d'un acide organique ou inorganique non toxique, physiologiquement acceptable et en particulier pharmaceutiquement acceptable, par exemple l'anion ou un équivalent d'anion d'un des acides cités plus haut utile pour la formation des sels d'addition. Q⁻ peut être par exemple un des anions (ou équivalent d'anion) d'un groupe choisi parmi chlore, sulfate, phosphate, acétate, trifluoroacétate, citrate, benzoate, maléate, fumarate, tartrate, méthanesulfonate et para-toluènesulfonate.

Les sels des composés de formule (I) peuvent être obtenus par les méthodes ordinaires connues de l'homme du métier, par exemple en combinant un composé de formule (I) avec un acide organique ou inorganique ou une base dans un solvant ou un dispersant ou à partir d'un autre sel par échange de cation ou d'anion.

L'invention inclut également tous les sels des composés de formule (I) qui, à cause de leur faible acceptabilité physiologique, ne sont pas directement utilisable comme médicament, mais sont utilisables comme produits intermédiaires pour mettre en oeuvre des modifications chimiques ultérieures au niveau des composés de formule (I) ou comme produits de départ pour la préparation de sels physiologiquement acceptables.

La présente invention inclut également tous les solvates des composés de formule (I) par exemples les hydrates, les solvates formés avec les alcools, et tous les dérivés des composés de formule (I), par exemple les esters, prodrogues et autres dérivés physiologiquement acceptables, ainsi que les métabolites des composés de formule (I).

L'invention se rapporte également aux prodrogues des composés de formule (I) qui peuvent être transformées en composés de formule (I) in vivo dans les conditions physiologiques. Les prodrogues des composés de formule (I), à savoir les dérivés chimiquement modifiés des composés de formule (I) afin d'obtenir des propriétés améliorées de manière désirée, sont connus de l'homme du métier.

Pour avoir plus d'information sur le type de prodrug envisagé dans la présente invention, on peut citer les ouvrages suivants : Fleicher et al., Advanced Drug Delivery Review 19 (1996) 115-130; Design of prodrugs, H. Bundgaard, Ed., Elsevier, 1985; H. Bungaard, Drugs of the Future 16 (1991) 443; Saulnier et al. Bioorg. Med. Chem. Lett. 4 (1994) 1985; Safadi et al. Pharmaceutical Res. 10 (1993) 1350. Parmi les prodrugs appropriées des composés de formule (I) on peut citer :
- les prodrogues sous forme d'esters des groupes carboxyliques, sulfonique ou phosphonique, lorsque, par exemple, Ar³ est substitué respectivement par un groupement -CO₂H, -SO₃H ou -PO₃H.
- les prodrogues sous forme d'acyle et de carbamate pour les groupes contenant un azote acylable tel que les groupes amino ou guanidine.
- les prodrogues sous forme de dérivés quaternaires d'azote cyclique ou non (benzyl substitué).

Dans les prodrogues acylés ou sous forme de carbamate, une ou plusieurs fois, par exemple deux fois, un atome d'hydrogène situé sur l'atome d'azote est remplacé par un groupe acyle ou carbamate. Parmi les groupes acyles ou carbamates préférés, on peut citer les groupes R₁₂CO-, R₁₃OCO-, dans lesquels R₁₂ est un hydrogène ou un radical (C₁-C₁₈)-alkyle, (C₃-C₁₄)-cycloalkyle, (C₃-C₁₄)-cycloalkyle- (C₁-C₈) -alkyle, (C₅-C₁₄)-aryle, dans lequel 1 à 5 atomes de carbone peuvent être remplacés par des hétéroatomes tels que N,O,S ou (C₅-C₁₄)-aryle-(C₁-C₈)alkyle, dans lequel 1 à 5 atomes de carbone dans la partie aryle peuvent être remplacés par des hétéroatomes tels que N,O,S et R₁₃ à les mêmes valeurs que R₁₂ à l'exception d'hydrogène.

Bien entendu, Ar¹, Ar², Ar³, R¹, R², R³, R⁴, R⁵, R⁶, R⁷, R⁸, R⁹, R¹⁰, R¹¹, R¹² et R¹³ peuvent adopter les définitions ci-dessus de manière indépendantes les unes par rapport aux autres.

Parmi les définitions concernant la formule (I), on peut citer les valeurs préférées suivantes :
Ar¹ et Ar³ représentent des groupements phényles,
Ar¹ représente un groupement phényle et Ar³ représente un hétérocycle
A est de préférence un groupement méthylène,
B est de préférence un groupement - CH=CH-CH₂- ou - (cyclopropyle)-CH₂-, les dits groupements étant non substitués ou substitués par un ou plusieurs halogènes ou (C₁-C₄)-alkyle,
R¹ est de préférence un atome d'hydrogène ou un groupement méthyle ou éthyle non substitué ou substitué par fluor, -OH, -NH₂, (C₁-C₈)-alkyloxy, (C₁-C₈)-alkylamino, ou di-(C₁-C₈)-alkylamino, pyrrolidino ou 2-oxo-pyrrolidino.
R² et R³ sont de préférence des atomes d'halogène
R⁴ est de préférence un atome d'hydrogène
R⁶ est de préférence un atome d'hydrogène
R⁵ et R⁷ représentent de préférence hydrogène
R⁸, R⁹ et R¹⁰ représentent de préférence hydrogène, CN, halogène, -CF₃, -OCF₃, OH, -SO₃H, -P(O)(OH)₂, Carboxy, -OSO₃H, -OPO₃H, -NH₂, (C₁-C₆)-alkyle, un radical hétérocyclique saturé ou insaturé non aromatique, amino-(C₁-C₆)-alkyle, hydroxy-(C₁-C₆) -alkyle, (C₁-C₆)-alkyloxy, (C₁-C₆)-alkylamino- (C₁-C₆)-alkyloxy, (C₁-C₆)-alkyloxycarbonyle, (C₁-C₆)-alkylcarbonyle, (C₁-C₆)-alkylaminocarbonyle, (C₁-C₆)-alkylamino, di-(C₁-C₆)-alkylamino ou di-(C₁-C₆)-alkylamino-(C₁-C₆)-alkyloxy, les dits radicaux alkyles ou hétérocycles étant non substitués ou substitués par halogène, OH, SO₃H, P(O)(OH)₂, oxo, Carboxy, -OSO₃H, -OPO₃H₂, -NH₂, phényle, (C₁-C₆)-alkyle, (C₁-C₆) - alkyloxy, hydroxy-(C₁-C₆)-alkyle, (C₁-C₆)-alkylamino ou di-(C₁-C₆)-alkylamino.

L'invention a plus particulièrement pour objet les composés de formule (I) tels que définis plus haut dans lesquels A est un groupement -CH₂-, B est un groupement - CH=CH-CH₂- ou - Cyclopropyle-CH₂-, Ar¹ représente un phényle et Ar² représente un phénylène ainsi que leurs sels d'addition physiologiquement acceptables.

L'invention a plus particulièrement pour objet les composés de formule (I) tels que définis plus haut répondant à la structure : 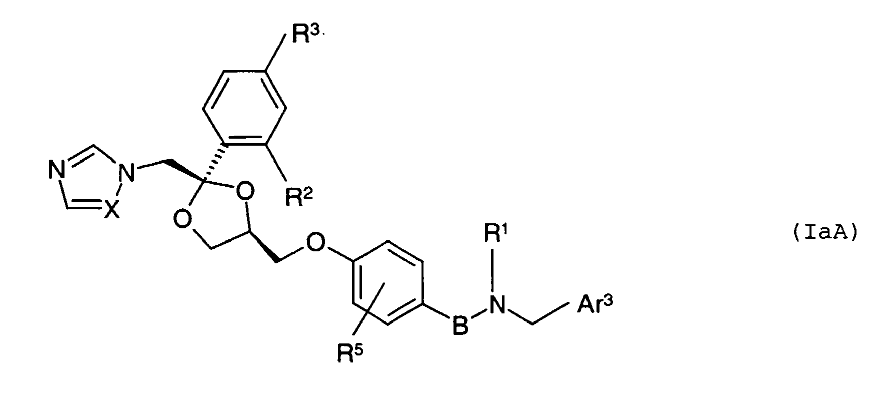 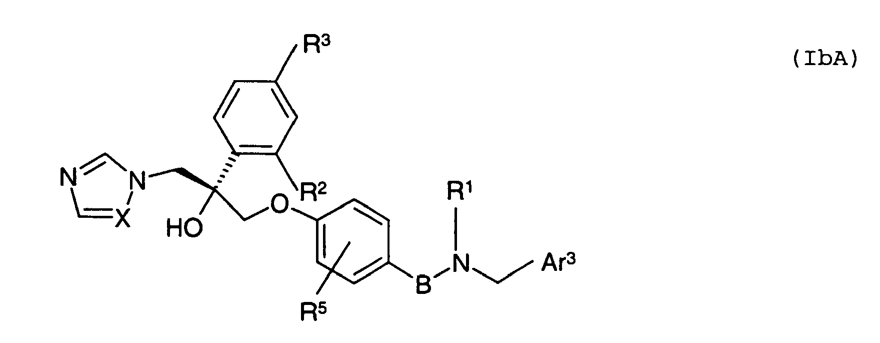 dans lesquelles, B, X, Ar³, R⁵ et R¹ sont tels que définis plus haut et R² et R³ représentent un atome de chlore ou de fluor ainsi que leurs sels d'addition physiologiquement acceptables.

L'invention a plus particulièrement pour objet les composés de formule (I) ou (IA) telles que définis plus haut dans lesquelles R₂ et R₃ sont des atomes de fluor ou de chlore, X représente CH ou N et Ar³ représente un groupement phényle non substitué ou substitué par R⁸ tel que défini précédemment, ainsi que leurs sels d'addition physiologiquement acceptables.

L'invention a tout particulièrement pour objet les composés de formule (I) ou (IA) telle que définies précédemment dans lesquelles, R¹ est un atome d'hydrogène ou un groupement méthyle, ou éthyle non substitué ou substitué par un groupement F, OH, NH₂, (C₁-C₆)-alkyloxy, (C₁-C₆)-alkylamino, di-(C₁-C₆)-alkylamino, pyrrolidino ou 2-oxo-pyrrolidino ainsi que leurs sels d'addition physiologiquement acceptables.

L'invention a tout particulièrement pour objet les composés de formule (IA) telles que définies précédemment dans laquelle Ar³ est un phényle non substitué ou substitué par R⁸ représentant un radical -Cl, -F, CN, -CF₃, -OCF₃, -OH, -NH₂, (C₁-C₆)-alkyloxy, (C₁-C₆)-alkylamino, di-(C₁-C₆)-alkylamino ou un hétérocycle choisi parmi :

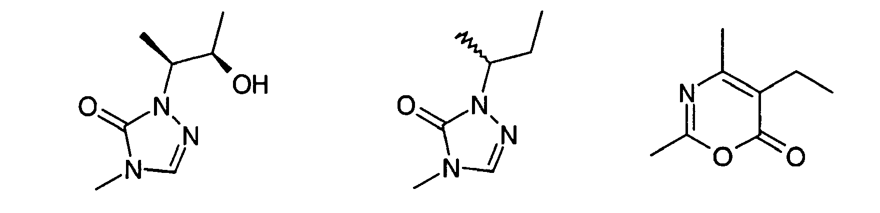

L'invention a tout particulièrement pour objet les composés suivants :
- Alpha-(2,4-dichlorophényl)-alpha-[[4-[3-[[méthyl(phenylmethyl)]amino]]-1(E)-propenyl]phenoxy]méthyl]-1H-imidazol-1-éthanol
- Alpha-(2,4-dichlorophényl)-alpha-[[4-[3-[[méthyl(1-naphtalènylméthyl)]amino]-1(E)-propényl]phenoxy]méthyl]-1H-imidazol-1-éthanol

L'invention a également pour objet un procédé de préparation des composés de formule (Ia) ou (Ib) caractérisé en ce que l'on soumet un composé de formule (IIa) ou (IIb) : 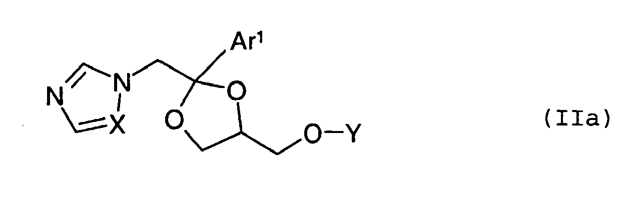 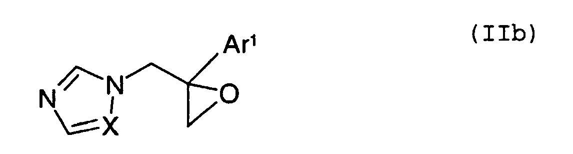 dans lesquelles Y représente un groupe partant après substitution nucléophile tel que mésylate ou tosylate et les autres substituants conservent leur signification précédentes à l'action, en présence d'une base, d'un composé de formule (III)

HO-Ar²-B-N (R¹) -A-Ar³ (III)

dans laquelle Ar², A,R¹,B et Ar³ conservent leur précédente signification, pour obtenir le composé de formule (Ia) ou (Ib) correspondant.

Cette réaction s'effectue dans les conditions classiques de substitution nucléophile du type R-OH + R'-OTs --> R-O-R' connues de l'homme du métier, Ts étant un groupe Tosyle. La base utilisée peut être notamment l'hydrure de sodium et le solvant peut être le DMF.

Les composés de formule (II) utilisés comme produits de départs sont des produits connus d'une façon générale, notamment lorsque Ar¹ est un phényle. Ils peuvent être préparés selon le procédé indiqué dans J. Med. Chem. (1979) 22(8) 1003.

Certains composés de formule (III) (R¹ = Me) sont aisément accessibles. Ils peuvent être préparés comme indiqué dans le schèma ci-dessous ou dans la partie expérimentale. 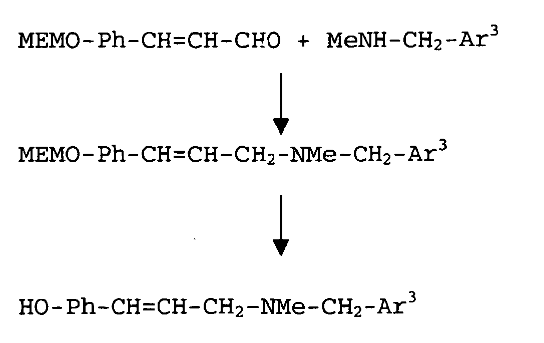 A titre de variante de procédé, on fait réagir le composé de formule (IIa) ou (IIb) avec un aryle de formule (III') HO-C₄H₆-CHO en présence d'une base, le phényle étant non substitué ou substitué par R⁵, afin d'obtenir un composé de formule (IVa) ou (IVb): 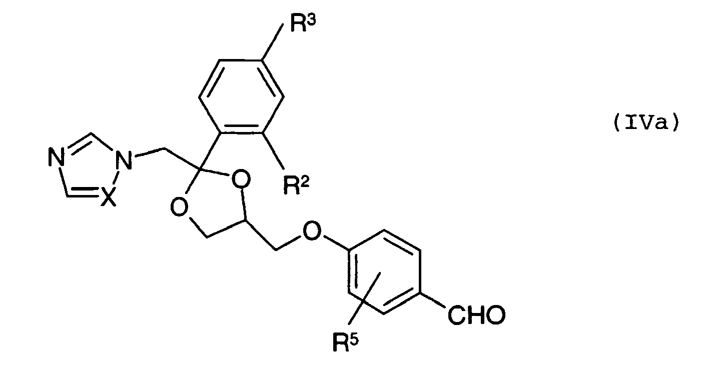 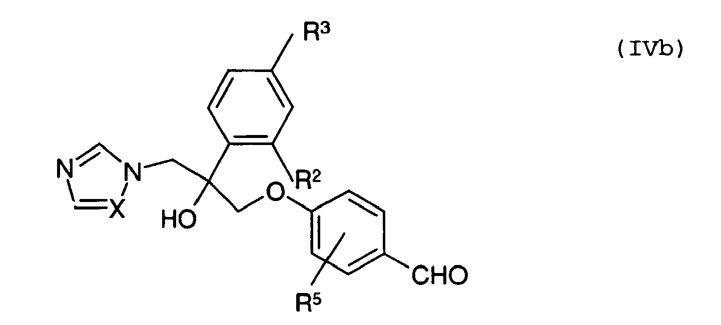 que l'on fait réagir successivement
a) avec un phosphorane du type (EtO)₂POCH₂CO₂Et pour obtenir l'ester insaturé correspondant,
b) avec un agent de réduction pour obtenir l'alcool correspondant
c) avec un agent d'oxydation pour former l'aldéhyde correspondant
d) avec un agent réducteur tel que NaBH₃CN, afin d'obtenir les composés de formule (Ia) ou (Ib)

Les composés de départs de formule (IVa) ou (IVb) peuvent être préparés selon des procédés décrits dans la littérature ou encore sont accessibles par analogie. La préparation des composés de formule (II) est décrite dans Eur. J. Med. Chem. (1995) 30, 617-626 ou J. Heterocyclic Chemistry (1990), 27 2053, étant entendu que la présente invention n'est pas restreinte à ces synthèses ou à ces produits de départ. Il n'y a pas de difficulté majeure pour l'homme du métier de prévoir des modifications des synthèses décrites dans notre demande pour la préparation d'autres composés de formule (I) selon l'invention.

Les composés de formule (I) sont des composés ayant une activité pharmacologique et peuvent ainsi être utilisés à titre de médicaments notamment comme antifongiques

La présente invention a donc pour objet les composés de formule (I) et/ou leurs sels physiologiquement acceptables et/ou leurs prodrugs à titre de médicament.

Les composés de formule (I) ainsi que leurs sels physiologiquement acceptables et leurs prodrugs peuvent être administrés aux animaux, de préférence aux mammifères et en particulier aux êtres humains comme médicaments à titre thérapeutique ou prophylactique.

Les composés de formule (I) présentent d'intéressantes propriétés antifongiques. Ils sont notamment actifs sur *Candida albicans* et autres *Candidas* comme *Candida glabrata, krusei, tropicalis, pseudotropicalis* et *parapsilosis,* sur *Aspergillus, Aspergillus flavus, Aspergillus niger, Cryptococcus néoformans, Microsporum canis, Trichophyton rubrun, Trichophyton mentagrophyte.*

Les composés de formule (I) peuvent être utilisés en tant que médicament chez l'homme ou l'animal, pour lutter notamment contre les candidoses digestives, urinaires, vaginales ou cutanées, les cryptococcoses, par exemple les cryptococcoses neuroméningées, pulmonaires ou cutanées, les aspergilloses bronchopulmonaires et pulmonaires et les aspergilloses invasives de l'immunodéprimé.

Les composés selon l'invention peuvent être utilisés également dans la prévention des affections mycosiques chez les déprimés immunitaires congénitaux ou acquis.

Les composés de l'invention ne sont pas limités à une utilisation pharmaceutiques. Ils peuvent être également utilisés comme fongicides dans d'autres domaines que pharmaceutiques.

L'invention a donc pour objet à titre de médicaments antifongiques, les composés de formule (Ia) ou (Ib).

L'invention a également pour objet l'application d'entités chimiques présentant à une extrémité les groupements suivants : 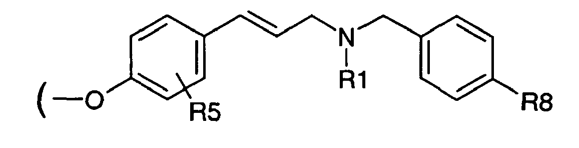 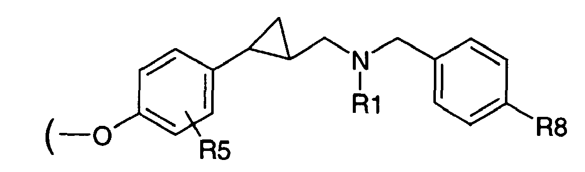 et à l'autre extrémité un groupement pharmacophore ayant une activité fongicide, tel que défini précédemment, pour la préparation de médicaments présentant une activité antifongiques.

Les composés selon l'invention peuvent être administrés tels quels ou en mélange avec un ou plusieurs autres composés de formule (I) ou encore sous la forme d'une préparation pharmaceutique (composition pharmaceutique) qui permet une administration entérale ou parentérale et qui renferme à titre de composé actif une dose efficace d'au moins un composé de formule (I) et/ou ses sels physiologiquement acceptables et/ou ses prodrugs ainsi que des supports et/ou additifs courants et pharmaceutiquement inertes.

Les compositions pharmaceutiques selon l'invention permettent une administration entérale ou parentérale qui renferment à titre de composé actif une dose efficace d'au moins un composé de formule (I) et/ou ses sels physiologiquement acceptables et/ou ses prodrugs ainsi qu'un ou plusieurs supports pharmaceutiquement inertes, et/ou un ou plusieurs additifs usuels.

L'invention a donc pour objet les compositions pharmaceutiques renfermant un composé de formules (Ia) ou (Ib) tel que défini précédemment ainsi qu'un vehicule.

Les médicaments peuvent être administrés oralement, par exemple sous forme de pilule, de comprimés, de comprimés enrobés, de pelliculés, de granules, de gélules et capsules molles, de solutions, de sirops, d'émulsion, de suspension ou de mélange d'aérosol.

L'administration peut cependant être effectuée par voie rectale, par exemple sous forme de suppositoire, par voie parentérale, par exemple sous forme de solutions injectables ou d'infusions, de microcapsules ou d'implants, par voie percutanée, par exemple sous la forme de pommade, de solutions, de pigments ou de colorants, par voie transdermique sous forme de patches ou par d'autres voies telles que sous la forme d'aérosol ou de spray nasal.

Les compositions pharmaceutiques selon l'invention sont préparées selon des méthodes connues en soi, des supports organiques ou inorganiques, pharmaceutiquement inertes étant additionnés aux composés de formule (I) et/ou leurs sels physiologiquement acceptables et/ou leurs prodrugs.

Pour la production de pilule, de comprimés, de comprimés enrobés et de capsule en gélatine dure, il est possible d'utiliser par exemple, du lactose, de l'amidon de maïs ou ses dérivés, du talc, de l'acide stéarique ou ses sels, etc. Les supports convenables pour des capsules en gélatine molle ou pour les suppositoires sont par exemple les graisses, les cires les polyols semi-solides ou liquides, les huiles naturelles ou modifiées etc. Les véhicules appropriés pour la préparation de solutions, par exemple les solutions injectables, les émulsions ou les sirops sont par exemple l'eau, les alcools, le glycérol, les polyols, le sucrose, les sucres invertis, le glucose, les huiles végétales, etc. Les supports convenables pour les microcapsules ou les implants sont par exemple les copolymères d'acide glyoxilique et d'acide lactique. Les préparations pharmaceutiques contiennent normalement de 0,5% à 90% en poids de composés de formule (I) et/ou leurs sels physiologiquement acceptables.

En plus des principes actifs et des supports, les préparations pharmaceutiques peuvent contenir des additifs tels que par exemple des diluants, des désintégrants, des liants, des lubrifiants, des agents mouillant, des stabilisants, des émulsifiants, des préservateurs, des agents sucrant, des colorants des agents de flaveurs ou des aromatisants, des épaississants, des agents tampons, et aussi des solvants ou des solubilisants ou des agents pour obtenir un effet retard et également des sels pour modifier la pression osmotique, des agents d'enrobage ou des antioxydants. Elles peuvent également contenir deux ou plusieurs composés de formule (I) et/ou leurs sels physiologiquement acceptables et/ou leurs prodrugs. En outre, en plus d'au moins un ou plusieurs composés de formule (I) et/ou leurs sels physiologiquement acceptables et/ou leurs prodrugs, ils peuvent contenir au moins un ou plusieurs autres principes actifs utilisables à titre thérapeutique ou prophylactique.

Les préparations pharmaceutiques (compositions pharmaceutiques) renferment normalement de 0,2 à 500 mg, et de préférence de 1 à 200 mg de composé de formule (I) et/ou leurs sels physiologiquement acceptables et/ou leurs prodrugs.

La présente invention a donc plus particulièrement pour objet un composé de formule (I) et/ou ses sels physiologiquement acceptables et/ou ses prodrugs tels que définis plus haut à titre de médicament ayant une activité antifongique.

La présente invention a également pour objet l'utilisation des composés de formule (I) et/ou leurs sels physiologiquement acceptables et/ou leurs prodrugs tels que définis plus haut pour la préparation de médicaments antifongiques.

Lorsqu'on utilise les composés de formule (I), les doses peuvent varier à l'intérieur de limites larges et doivent être fixées en fonction de la personne à traiter. Ceci dépend par exemple du composé employé ou de la nature et de la sévérité de la maladie à traiter et si on se trouve dans des conditions graves ou chronique ou si on met en oeuvre un traitement prophylactique.

Dans le cas d'une administration par voie orale, la dose quotidienne varie en général de 0,01 à 100mg/kg et de préférence de 0,1 à 50 mg/kg, en particulier de 0,1 à 5 mg/kg.

Dans le cas d'une administration par voie intraveineuse, la dose quotidienne varie approximativement de 0,01 à 100 mg/kg et de préférence de 0,05 à 10 mg/kg.

La dose quotidienne peut être divisée, en particulier dans le cas de l'administration de grande quantité de principe actif, en plusieurs, par exemple 2,3 ou 4 parts. Le cas échéant, en fonction du comportement individuel, il peut être nécessaire d'administrer les différentes doses de manière croissante ou décroissante.

Les composés de formule (I) et leurs sels peuvent également être utilisés comme intermédiaires pour la préparation d'autres composés, en particulier d'autres agents actifs, qui sont accessibles à partir des composés de formule (I), par exemple par modification ou introduction de radicaux ou de groupes fonctionnels.

### Exemples

Les produits ont été identifiés par spectre de masse (MS), infrarouge (IR) et/ou spectre RMN. Les composés ont été purifiés par chromatographie en phase normale (notamment en présence du mélange CH₂Cl₂/MeOH) ou en phase inverse (en présence d'acide acétique ou trifluoroacétique). Les composés de formule (I) purifiés en utilisant un éluant qui contient par exemple de l'acide trifluoroacétique, et qui ensuite sont séchés ou dans lesquels, lors de la dernière étape de synthèse, par exemple de l'acide trifluoroacétique a été utilisé pour éliminer un groupe protecteur tert-butyl, contiennent parfois, en fonction de la manière dont le produit a été séché, l'acide provenant de l'éluant ou de la dernière étape de synthèse et donc se trouvent partiellement ou complètement sous la forme du sel de l'acide utilisé, par exemple sous la forme d'un sel d'acide acétique ou trifluoroacétique. Ils peuvent également être plus ou moins hydratés.

### Abbrévations/noms chimiques éventuellement employés :

AcOEt : acétate d'éthyle ; DMF : diméthylformamide ; HOBt : 1-hydroxybenzotriazole hydrate, MeOH : méthanol ; TEA : triéthylamine ; TFA : acide trifluoroacétique ; THF : tétrahydrofurane ; MCPBA : acide méta-chloroperoxybenzoique ; DBU : 1,8-diazabicyclo[5.4.0]undec-7-ene; APTS : acide paratoluènesulfonique ; DPPA : diphénylphosphorylazide ; DMSO : diméthylsulfoxyde ; Pd/C Palladium sur charbon ; Boc : terbutoxycarbonyl ; CBz : benzyloxycarbonyl ; DCC 1,3-dicyclohexylcarbodiimide ;
IR : Infrarouge ; RMN : Résonnance Magnétique Nucléaire ; SM : Spectre de Masse ; ESP : Electrospray mode positif ; ep : Épaulement ; F : fort ; s : singulet ; d : doublet ; t : triplet ; quad : quadruplet ; quint : quintuplet ; 1 : large ; m : multiplet ou massif; J : constante de couplage ; Rf : facteur de rétention (chromatographie).

Les spectres RMN ci-dessous ont été interprétés et les hydrogènes aromatiques sont identifiées ainsi

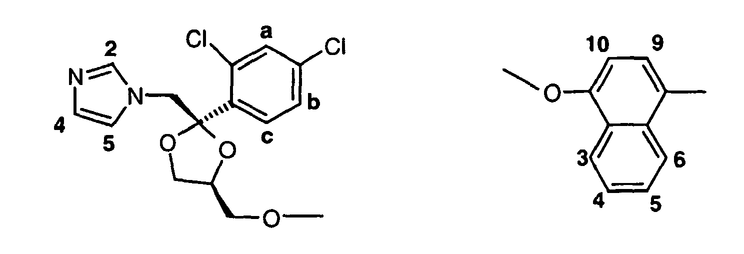

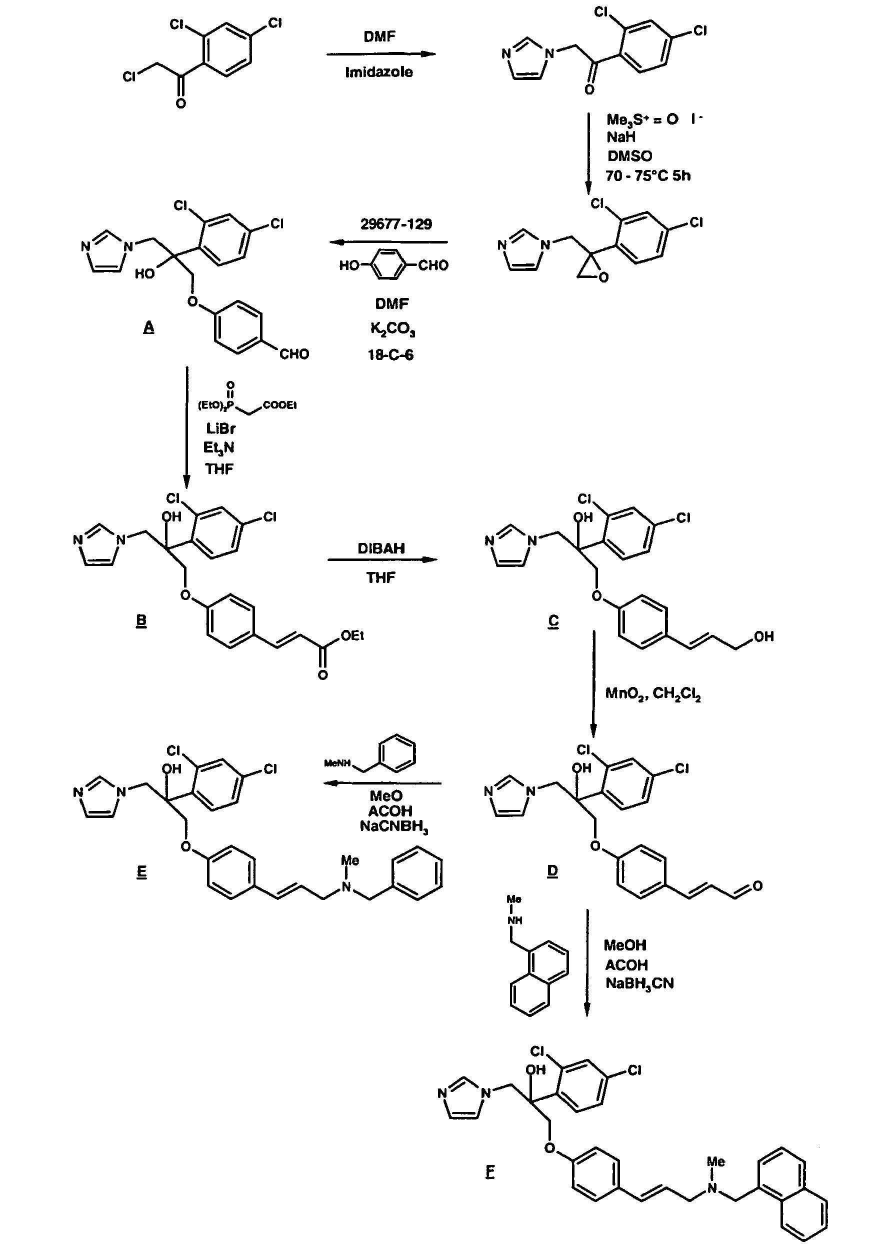

### Exemple 1 : Alpha-(2,4-dichlorophényl)-alpha-[[4-[3-[[méthyl (phénylméthyl)]amino]]-1(E)-propényl]phénoxy]méthyl]-1H-imidazol-1-éthanol

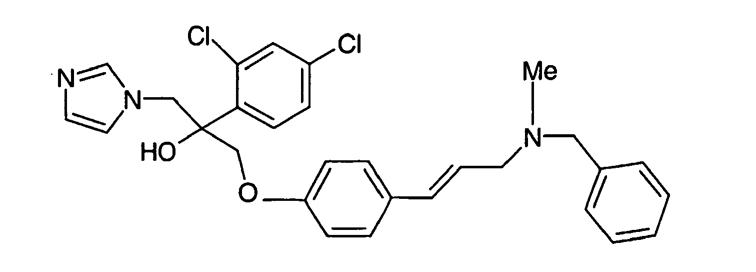

### Stade a : A → B

On introduit le bromure de lithium (1,38 g) à une solution de triéthylphosphonoacétate (3,16 ml) dans le THF (13 ml). On agite pendant 10 minutes à température ambiante. Puis on ajoute le triéthylamine (2,24 ml) et on agite encore pendant 10 minutes à température ambiante. Puis on ajoute rapidement une suspension d'aldéhyde IVb (X = CH, R₂ = R₃ = Cl, 1,56 g) dans 15 ml de THF. Après avoir agité pendant 24 heures à 25°C, on chauffe à 55°C pendant 4h. On refroidit, on extrait par dichlorométhane puis on lave avec de l'eau. On sèche la phase organique (MgSO₄), on filtre et on évapore pour obtenir 3,6 g du produit brut que l'on purifie par chromatographie sur silice en éluant avec le mélange dichlorométhane/ méthanol (96/4). On obtient le produit attendu.
CCM (silice, CH₂Cl₂-MeOH, 96 :4) Rf = 0,25.

### Stade b : B → C

On refroidit à 0°C une solution du produit du stade a (1,19 g, 2,6 mmol) dans le THF (11 ml) puis on ajoute une solution de DIBAH (3,4 ml, 1,5 M) dans le toluène en maintenant la température inférieure à 10°C. Après avoir agité pendant 2 heures, on laisse revenir à 15°C et on ajoute une solution de DIBAH (8,5 ml, 1,5 M) dans le toluène en maintenant la température à 15°C. On agite à température ambiante pendant 48 heures. Puis on refroidit la solution à 0°C et on verse 5 ml de solution saturée de tartrate double de sodium et potassium. On agite pendant 1 heure à température ambiante. Après avoir dilué avec 15 ml d'eau, on extrait par 3 x 50 ml de CH₂Cl₂ on sèche sur MgSO₄, on filtre et on évapore à sec pour obtenir le produit brut (0,83 g). On purifie par chromatographie sur silice en éluant avec le mélange dichlorométhane / méthanol (96/4) pour obtenir le produit attendu.
CCM (silice, CH₂Cl₂-MeOH, 96 :4) Rf = 0,15.

### Stade c : C → D

A une solution du produit du stade b (0,52 g) dans le dichlorométhane (12 ml), on introduit en une fois MnO₂ (1,07 g) et on agite pendant 48 heures à température ambiante. On filtre sur Clarcel flow M, on lave avec CH₂Cl₂, on évapore à sec pour donner le produit cherché (0,46 g).
CCM (silice, CH₂Cl₂-MeOH, 96 : 4) Rf = 0,3.

### Stade d : D → E

A une solution du produit du stade c (0,125 g) dans le méthanol (1,2 ml) à température ambiante, on ajoute du N-méthylbenzylamine (0,04 g) et de l'acide acétique (23 µl). On agite pendant 10 minutes avant d'ajouter NaBH₃CN (20 mg). Après avoir agité pendant 4 heures, on ajoute de l'eau et du dichlorométhane. On amène à pH 8 avec une solution d'ammoniaque concentré. On extrait avec du dichlorométhane, on sèche sur MgSO₄, on filtre et on évapore à sec sous vide pour donner le produit brut (0,17 g).

On purifie par chromatographie sur silice en éluant avec le mélange dichlorométhane/méthanol (96/4) pour obtenir le produit attendu (38 mg, RU 831624).
CCM (silice, CH₂Cl₂-MeOH-NH4OH, 96 :4 :0,1) Rf = 0,15.

### Exemple 2 : Alpha-(2,4-dichlorophényl)-alpha-[[4-[3-[[méthyl(1-naphtalènylméthyl)]amino]-1(E)-propényl] phénoxy]méthyl]-1H-imidazol-1-éthanol

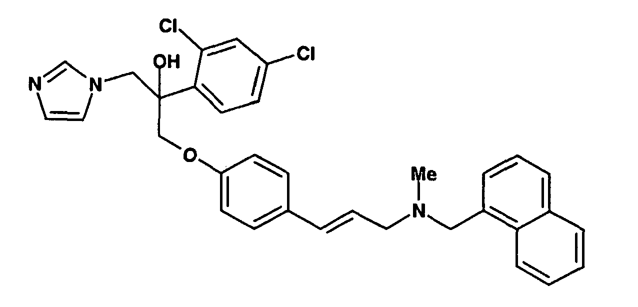

### Stade d' : D → F

De manière similaire à l'exemple 1, on a préparé le produit ci-dessus.
CCM (silice, CH₂Cl₂-MeOH, 95 :5) Rf = 0,3.

### Compositions pharmaceutiques

On a préparé des composés renfermant

| | |
|---|---|
| Produit de l'exemple 1 | 50 mg |
| Excipient q.s.p. | 1 g |

Détail de l'excipient: amidon, talc, stéarate de magnésium.

### Activité biologique

### 1) Activité antifongique des composés selon l'invention.

On utilise des souris femelles pesant de 18 à 22 g. On leur administre dans la veine de la queue une quantité de *Candida albicans* 44858 à raison de 10⁶CFU par souris (CFU : unité formant des colonies). On sépare les souris en 5 lots de 5 souris et on les traite de la façon suivante :
Une heure après l'infection
   - groupe 1 : les souris sont traitées avec le produit P 25 mg/kg par voie orale
   - groupe 2 : les souris sont traitées avec le produit P par voie intrapéritonéale à raison de 25 mg/kg
   - groupe 3 : les souris sont traitées avec le fluconazole (25 mg/kg par voie orale).
   - groupe 4 : les souris sont traitées avec le fluconazole (25 mg/kg par voie intrapéritonéale).
   - groupe 5 : les souris ne reçoivent aucun traitement antifongique.

Pendant une période de 22 jours, on compte les souris mortes.

### 2) Concentration minimale inhibitrice (CMI)

Des cellules de *Candida albicans* sont préparées comme indiqué dan Journal of Antimicrobial chemotherapy 38, 579-587, lavées 3 fois avec une solution 0,1 M de phosphate et utilisées immédiatement pour déterminer la concentration minimale inhibitrice (CMI).

Les CMI sont déterminés par la modification d'une plaque microtitré selon la méthode standard du Comité National des standards cliniques de laboratoire.

On utilise comme milieu RPMI-1640, et de la L-glutamine tamponnée à pH7 avec une solution 0,15 M de MOPS (acide 3-[N-morpholino]propane sulfonique). On ajoute les cellules de *Candida albicans* (1,5 X 10³ cellules/ml) dans les puits d'une plaque de 96 puits contenant RPMI-1640 et les dilutions d'agents antifongiques. On fait la lecture des résultats 48 heures après incubation à 35°C et on détermine la CMI ou concentration minimale inhibitrice qui inhibe la croissance des cellules du *Candida albicans.*

### Concentration minimale fongicide

Après la lecture à 48 heures des CMI, on secoue les plaques et retire 10 µL d'aliquote des puits que l'on place sur des disques rectangulaires contenant du dextrose agar. Les plaques sont incubées pendant 48 heures à 35°C ; La concentration minimale fongicide et la concentration de l'agent antifongique à laquelle le nombre d'unité formant des colonies est zéro.

### Conclusion

Les composés selon l'invention décrits aux exemples 1 à 2 présentent une activité à < 100 µg/ml dans le test CMI.

## Revendications

1. Les composés de formule (Ia)et (Ib) 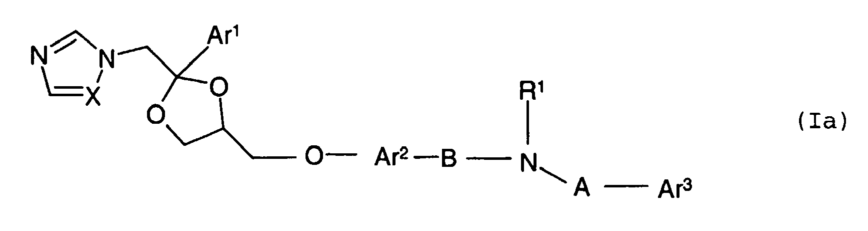 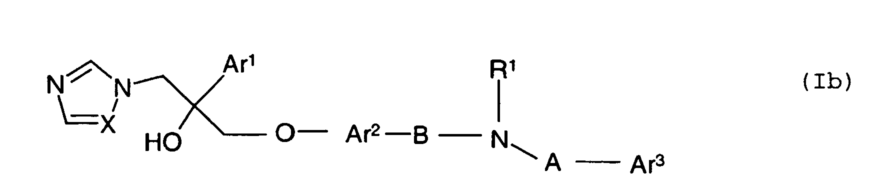 dans lesquelles
- X est un atome d'azote ou un groupement CH,
- Ar¹ représente un aryle carbocyclique ou hétérocyclique non substitué ou substitué par un ou plusieurs radicaux R², R³ ou R⁴
- Ar² représente un phénylène ou naphtylène non substitué ou substitué par un ou plusieurs radicaux R⁵, R⁶ ou R⁷
- Ar³ représente un aryle carbocyclique ou hétérocyclique non substitué ou substitué par un ou plusieurs radicaux R⁸, R⁹ ou R¹⁰
- A représente un radical (C₁-C₄)-alkylène,
- B représente un radical -CH=CH-(C₁-C₄)-alkylène- ou un radical -cyclopropylène-(C₁-C₄)-alkylène-, les dits radicaux cyclopropylène ou -CH=CH- étant non substitués ou substitués par un radical R² et/ou R³,
- R¹ représente un atome d'hydrogène, un groupement -SO₃H ou bien un radical (C₁-C₆)-alkyle non substitué ou substitué par un radical tel que défini pour R², ou un radical alkylène lié au group Ar³
- R², R³, R⁴, R⁵, R⁶, R⁷, R⁸, R⁹ ou R¹⁰ identiques ou différents, représentent fluor, chlore, brome, cyano, mono- bi- ou trihalogeno(C₁-C₈)alkyle, mono- bi- ou trihalogeno (C₁-C₈)-alkyloxy, hydroxy, nitro, carboxyle, formyle, -SO₃H, -OSO₃H, (R¹¹O)₂P(O)-, (R¹¹O)₂P(O)-O-, amino, (C₁-C₈)-alkylamino, di ((C₁-C₈) alkyl) amino, (C₅-C₁₄)-aryl-(C₁-C₆)-alkylènamino ou (C₅-C₁₄)-arylamino, (C₁-C₈)-alkyle, (C₅-C₁₄)-aryle, un hétérocycle éventuellement substitué par oxo, (C₅-C₁₄)-aryl-(C₁-C₆)alkyle, amino- (C₁-C₆)-alkyle, (C₁-C₈)-alkylamino- (C₁-C₆) -alkyle, di- ((C₁-C₈)alkyl) amino- (C₁-C₆)-alkyle, hydroxy-(C₁-C₆) alkyle, (C₁-C₆)-alkyloxy-(C₁-C₆) -alkyle, (C₁-C₈)-alkyloxy éventuellement interrompu par un bu plusieurs atomes d'oxygène, (C₅-C₁₄)-aryl-(C₁-C₆)-alkylènoxy, (C₅-C₁₄)-aryloxy, hydroxy-(C₁-C₆)alkylènoxy, (C₁-C₆)-alkyloxy-(C₁-C₆)alkylènoxy, amino-(C₁-C₆)-alkylènoxy, (C₁-C₆)-alkylamino-(C₁-C₆) -alkylènoxy, di ((C₁-C₈) -alkyl) amino- (C₁-C₆) -alkylènoxy, méthylènedioxy, (C₁-C₆)-alkyloxycarbonyle, (C₁-C₆)-alkylcarbonyle, (C₅-C₁₄) aryl- (C₁-C₆) -alkylènecarbonyl, (C₅-C₁₄)-aryl-carbonyle, (C₁-C₆)-alkylaminocarbonyle, (C₁-C₆)alkanoylamino, (C₁-C₆)-alkylsulfonylamino, (C₅-C₁₉)-arylsulfonylamino, (C₅-C₁₄) -aryl- (C₁-C₆) -alkylènesulfonylamino, (C₁-C₆)-alkylaminosulfonyle, (C₅-C₁₄)-aryl-(C₁-C₆)-alkylènaminosulfonyl, (C₁-C₆)-alkylsulfonyl, (C₅-C₁₄)-aryl-(C₁-C₈)-alkylènesulfonyle ou (C₅-C₁₄)-aryl-sulfonyle, les dits radicaux alkyles, aryles ou hétérocycles étant eux même non substitués ou substitués par un ou plusieurs groupements cités ci-dessus.
- R¹¹ représente hydrogène, (C₁-C₁₀)-alkyl, (C₆-C₁₄)-aryle ou (C₆-C₁₄) -aryl-(C₁-C₆)-alkyle,
sous toutes leurs formes stéréoisomères possibles et leurs mélanges,ainsi que leurs sels d'addition physiologiquement acceptables et leurs prodrogues sous forme d'esters des groupes carboxylique, sulfonique ou phosphonique, ou sous forme d'acyle et de carbonate pour les groupes contenant un acide acylable, ou sous forme de dérivés quaternaires d'azote cyclique ou non.

2. Les composés de formules (Ia) ou (Ib) tels que définis à la revendication 1, dans lesquels A est un groupement -CH₂-, B est un groupement - CH=CH-CH₂- ou -Cyclopropyle-CH₂- et Ar¹ représente un phényle et Ar² représente un phénylène-ainsi que leurs sels d'addition physiologiquement acceptables.

3. Les composés de formule (Ia)ou (Ib) tels que définis à la revendication 1 de structure : 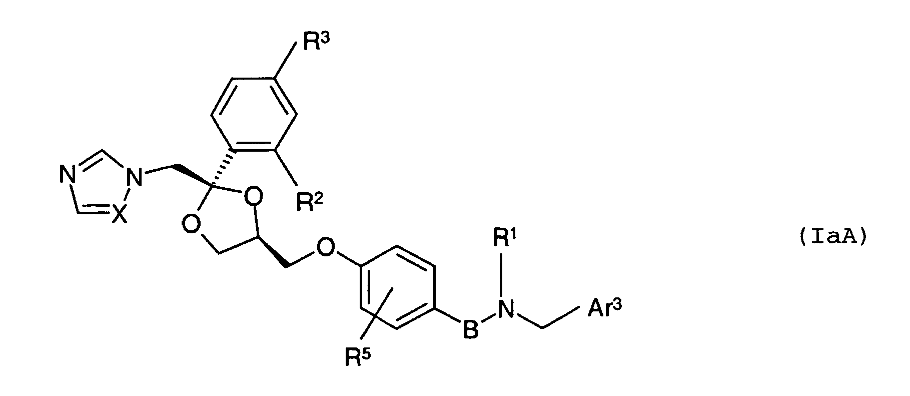 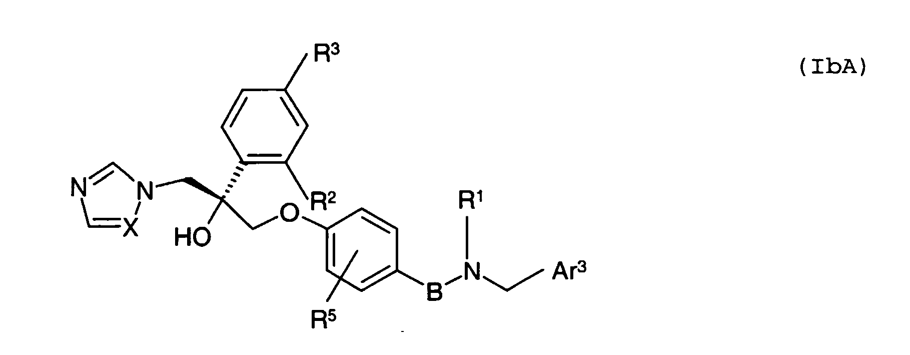 dans lesquelles, B, X, Ar³, R⁵ et R¹ sont tels que définis à la revendication 1 et R² et R³ représentent un atome d'halogène. ainsi que leurs sels d'addition physiologiquement acceptables.

4. Les composés de formules (Ia) (Ib) (IaA) ou (IbA) tels que définis à l'une quelconque des revendications 1 à 3 dans lesquels R₂ et R₃ sont des atomes de chlore, X représente CH ou N et Ar³ représente un groupement phényle non substitué ou substitué par R⁸ tel que défini à la revendication 1, ainsi que leurs sels d'addition physiologiquement acceptables.

5. Les composés de formule (Ia), (Ib), (IaA) ou (IbA) tels que définis à l'une quelconque des revendications 1 à 4 dans lesquelles, R¹ est un atome d'hydrogène ou un groupement méthyle, ou éthyle non substitué ou substitué par un groupement F, OH, NH₂, (C₁-C₆)-alkyloxy, (C₁-C₆)-alkylamino, di-(C₁-C₆)-alkylamino, pyrrolidino ou 2-oxo-pyrrolidino, ainsi que leurs sels d'addition physiologiquement acceptables.

6. Les composés de formules (Ia), (Ib), (IaA) ou (IbA) tels que définis à l'une quelconque des revendications 1 à 5 dans lesquelles Ar³ est un phényle non susbtitué ou substitué par R^{a} représentant un radical -Cl, -F, CN, -CF₃, -OCF₃, -OH, -NH₂, (C₁-C₆)-alkyloxy, (C₁-C₆)-alkylamino, ou di-(C₁-C₆)-alkylamino ou un hétérocycle choisi parmi : 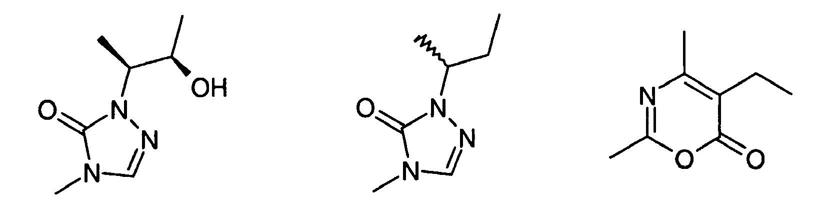 ainsi que leurs sels d'addition physiologiquement acceptables.

7. Les composés de formule (Ib) tels que définis à la revendication 1 dont les noms suivent :
- Alpha-(2,4-dichlorophényl)-alpha-[[4-[3-[[méthyl(phenylmethyl)]amino]]-1(E)-propenyl]phenoxy]méthyl]-1Himidazol-1-éthanol
- Alpha-(2,4-dichlorophényl)-alpha-[[4-[3-[[méthyl(1-naphtalènylméthyl)]amino]-1(E)-propényl]phenoxy]méthyl]-1H-imidazol-1-éthanol

8. Un procédé de préparation des composés de formule (Ia) ou (Ib) selon l'une quelconque des revendications 1 à 7 **caractérisé en ce que** l'on soumet un composé de formules (IIa) ou (IIb) 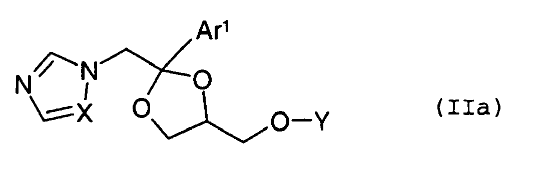 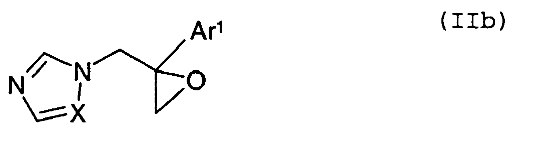 dans lesquelles Y représente un groupe partant après substitution nucléophile tel que mésylate ou tosylate et les autres substituants conservent leur signification précédentes à l'action, en présence d'une base, d'un composé de formule (III) :
HO-Ar²-B-N(R¹)-A-Ar³ (III)
dans laquelle Ar², A, R¹, B et Ar³ sont tels que définis à la revendication 1, pour obtenir le composé de formule (I) correspondant.

9. Un procédé de préparation des composés de formule (I) selon l'une quelconque des revendications 1 à 7 **caractérisé en ce que** l'on soumet un composé de formules (IIa) (IIb) avec un aryle de formule (III') HO-C₆H₄-CHO, en présence d'une base, le phénylène étant non substitué ou substitué par R⁵ afin d'obtenir un composé de formules (IVa) ou (IVb): 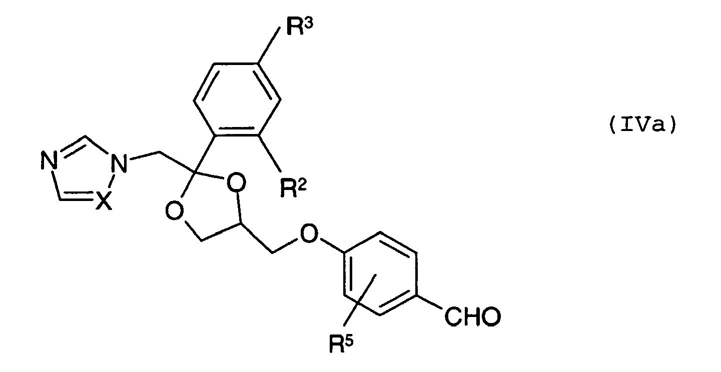 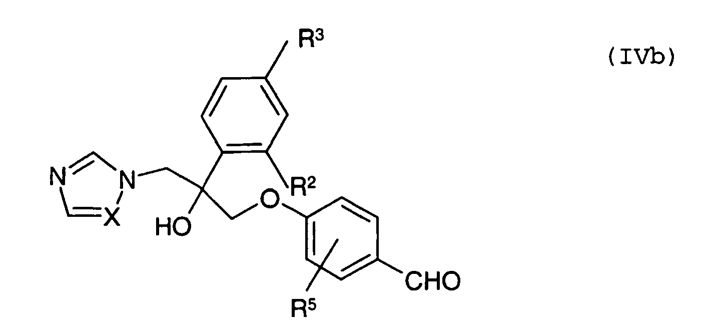 que l'on fait réagir successivement
a)avec un phosphorane du type (EtO)₂POCH₂CO₂Et pour obtenir l'ester allylique correspondant,
b)avec un agent de réduction pour obtenir l'alcool correspondant
c)avec un agent d'oxydation pour former l'aldéhyde correspondant
d) avec un agent réducteur tel que NaBH₃CN, afin d'obtenir les composés de formule (Ia) ou (Ib)

10. A titre de médicament les composés de formules (Ia) ou (Ib) tels que définis à l'une quelconque des revendications 1 à 7 ainsi que leurs sels d'addition pharmaceutiquement acceptables et/ou leurs prodrogues sous forme d'esters des groupes carboxylique, sulfonique ou phosphonique, ou sous forme d'acyle et de carbonate pour les groupes contenant un acide acylable, ou sous forme de dérivés quaternaires d'azote cyclique ou non.

11. Les composés de formules (Ia) ou (Ib) tels que définis à l'une quelconque des revendications 1 à 7 ainsi que leurs sels d'addition pharmaceutiquement acceptables et/ou leurs prodrogues sous forme d'esters des groupes carboxylique, sulfonique ou phosphonique, ou sous forme d'acyle et de carbonate pour les groupes contenant un acide acylable, ou sous forme de dérivés quaternaires d'azote cyclique ou non, pour leur utilisation à titre d'antifongique.

12. Application d'entités chimiques présentant à une extrémité les groupements suivants : 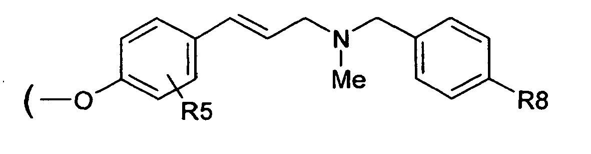 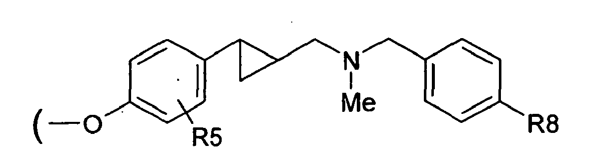 et à l'autre extrémité un groupement pharmacophore ayant une activité fongicide tel que défini à la revendication 1 et 2, pour la préparation de médicaments présentant une activité antifongiques.

13. Composition pharmaceutique renfermant au moins 1 composé de formule (Ia) ou (Ib) tels que définis à l'une quelconque des revendications 1 à 7 ainsi que leurs sels d'addition pharmaceutiquement acceptables et/ou leurs prodrogues sous forme d'esters des groupes carboxylique, sulfonique ou phosphonique, ou sous forme d'acyle et de carbonate pour les groupes contenant un acide acylable, ou sous forme de dérivés quaternaires d'azote cyclique ou non, et un véhicule pharmaceutiquement acceptables.

## Claims

1. Compounds of formulae (Ia) and (Ib) 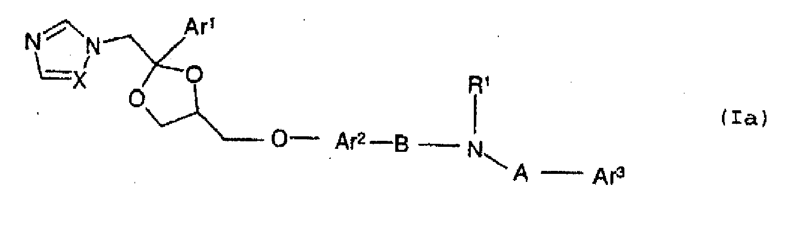 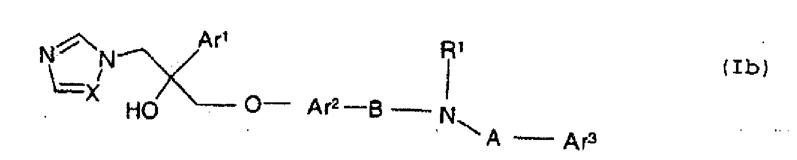 in which
- X is a nitrogen atom or a CH group,
- Ar¹ represents a carbocyclic or heterocyclic aryl that is unsubstituted or substituted with one or more radicals R², R³ or R⁴
- Ar² represents a phenylene or naphthylene that is unsubstituted or substituted with one or more radicals R⁵, R⁶ or R⁷,
- Ar³ represents a carbocyclic or heterocyclic aryl that is unsubstituted or substituted with one or more radicals R⁸, R⁹ or R¹⁰,
- A represents a (C₁-C₄)alkylene radical,
- B represents a -CH=CH-(C₁-C₄)alkylene- radical or a -cyclopropylene-(C₁-C₄)alkylene- radical, said cyclopropylene or -CH=CH- radicals being unsubstituted or substituted with a radical R² and/or R³,
- R¹ represents a hydrogen atom, an -SO₃H group or else a (C₁-C₆)alkyl radical that is unsubstituted or substituted with a radical as defined for R², or an alkylene radical bonded to the Ar³ group,
- R², R³, R⁴, R⁵, R⁶, R⁷, R⁸, R⁹ or R¹⁰, which may be identical or different, represent fluorine, chlorine, bromine, cyano, mono-, bi- or trihalo-(C₁-C₈)alkyl, mono-, bi- or trihalo(C₁-C₈)alkyloxy, hydroxyl, nitro, carboxyl, formyl, -SO₃H, -OSO₃H, (R¹¹O)₂P(O)-, (R¹¹O)₂P(O)-O-, amino, (C₁-C₈) alkylamino, di ((C₁-C₈) alkyl) amino, (C₅-C₁₄) aryl (C₁-C₆)-alkyleneamino or (C₅-C₁₄) arylamino, (C₁-C₈) alkyl, (C₅-C₁₄)aryl, a heterocycle optionally substituted with oxo, (C₅-C₁₄) aryl (C₁-C₆)alkyl, amino-(C₁-C₆)alkyl, (C₁-C₈)alkylamino(C₁-C₆)alkyl, di-((C₁-C₈)alkyl) amino (C₁-C₆)alkyl, hydroxyl-(C₁-C₆) alkyl, (C₁-C₆) alkyloxy(C₁-C₆) alkyl, (C₁-C₈)alkyloxy optionally interrupted with one or more oxygen atoms, (C₅-C₁₄) aryl (C₁-C₆)alkyleneoxy, (C₅-C₁₄)aryloxy, hydroxy(C₁-C₆)alkyleneoxy, (C₁-C₆) alkyloxy (C₁-C₆)alkyleneoxy, amino (C₁-C₆)-alkyleneoxy, (C₁-C₆) alkylamino(C₁-C₆) alkyleneoxy, di ((C₁-C₆) alkyl) amino (C₁-C₆) alkyleneoxy, methylenedioxy, (C₁-C₆)alkyloxycarbonyl, (C₁-C₆) alkylcarbonyl, (C₅-C₁₄) aryl (C₁-C₆) alkylene-carbonyl, (C₅-C₁₄) arylcarbonyl, (C₁-C₆) alkylaminocarbonyl, (C₁-C₆)alkanoylamino, (C₁-C₆) alkylsulphonylamino, (C₅-C₁₄)arylsulphonylamino, (C₅-C₁₄) aryl (C₁-C₆) alkylenesulphonylamino, (C₁-C₆)-alkylaminosulphonyl, (C₅-C₁₄)aryl (C₁-C₆)alkylene-aminosulphonyl, (C₁-C₆)alkylsulphonyl, (C₅-C₁₄)aryl-(C₁-C₈)alkylenesulphonyl or (C₅-C₁₄)arylsulphonyl, said alkyl, aryl or heterocycle radicals being themselves unsubstituted or substituted with one or more groups mentioned above,
- R¹¹ represents hydrogen, (C₁-C₁₀) alkyl, (C₆-C₁₄)aryl or (C₆-C₁₄)aryl(C₁-C₆)alkyl,
in any of their possible stereoisomeric forms and their mixtures, and also their physiologically acceptable addition salts and their prodrugs, in the form of esters of carboxylic, sulphonic or phosphonic groups, or in acyl and carbonate form for the groups containing an acid that can be acylated, or in the form of quaternary derivatives of cyclic or noncyclic nitrogen.

2. Compounds of formula (Ia) or (Ib) as defined in Claim 1, in which A is a -CH₂- group, B is a -CH=CH-CH₂- or -cyclopropyl-CH₂- group and Ar¹ represents a phenyl and Ar² represents a phenylene, and also their physiologically acceptable addition salts.

3. Compounds of formula (Ia) or (Ib) as defined in Claim 1, with the structure: 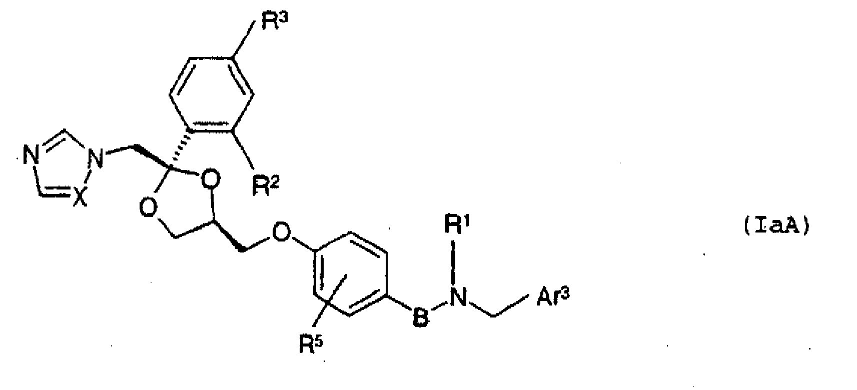 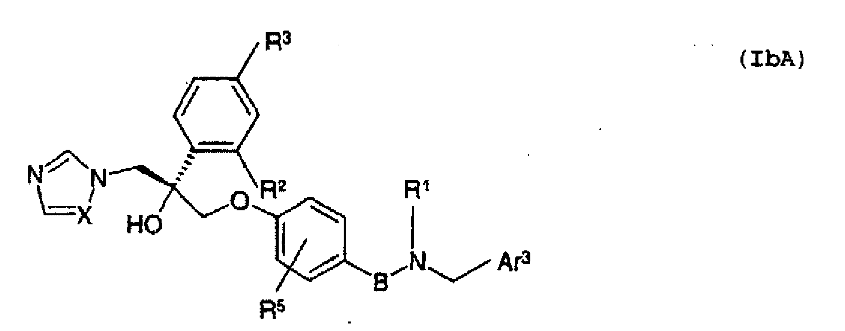 in which B, X, Ar³, R⁵ and R¹ are as defined in Claim 1, and R² and R³ represent a halogen atom, and also their physiologically acceptable addition salts.

4. Compounds of formula (Ia) , (Ib), (IaA) or (IbA) as defined in any of Claims 1, to 3, in which R² and R³ are chlorine atoms, X represents CH or N and Ar³ represents a phenyl group that is unsubstituted or substituted with R⁸ as defined in Claim 1, and also their physiologically acceptable addition salts.

5. Compounds of formula (Ia), (Ib), (IaA) or (IbA) as defined in any one of Claims 1 to 4, in which R¹ is a hydrogen atom or a methyl or ethyl group that is unsubstituted or substituted with an F, OH, NH₂, (C₁-C₆)alkyloxy, (C₁-C₆)alkylamino, di(C₁-C₆) alkylamino, pyrrolidino or 2-oxopyrrolidino group, and also their physiologically acceptable addition salts.

6. Compounds of formula (Ia), (Ib), (IaA) or (IbA) as defined in any one of Claims 1 to 5, in which Ar³ is a phenyl that is unsubstituted or substituted with R⁸ representing a -Cl, -F, CN, -CF₃, -OCF₃, -OH, -NH₂, (C₁-C₆)alkyloxy, (C₁-C₆)alkylamino or di(C₁-C₆)alkylamino radical, or a heterocycle chosen from: 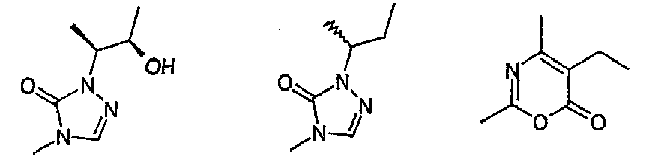 and also their physiologically acceptable addition salts.

7. Compounds of formula (Ib) as defined in Claim 1, the names of which follow:
- Alpha-(2,4-dichlorophenyl)-alpha-[[4-[3-[[methyl-(phenylmethyl)amino]]-1(E)-propenyl]phenoxy]methyl]-1H-imidazol-1-ethanol
- Alpha-(2,4-dichlorophenyl)-alpha-[[4-[3-[methyl(1-naphthalenylmethyl)amino]-1(E)-propenyl]phenoxy]-methyl]-1H-imidazol-1-ethanol.

8. Method for preparing the compounds of formula (Ia) or (Ib) according to any one of Claims 1 to 7, **characterized in that** a compound of formula (IIa) or (IIb) 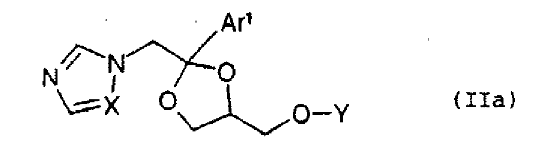 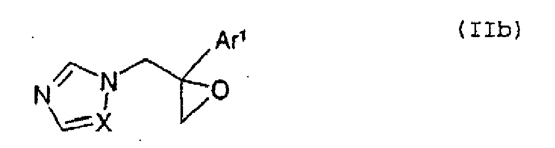 in which Y represents a leaving group after nucleophilic substitution, such as mesylate or tosylate, and the other substituents retain their previous meaning, is subjected to the action, in the presence of a base, of a compound of formula (III) :
HO-Ar²-B-N(R¹)-A-Ar³ (III)
in which Ar², A, R¹, B and Ar³ are as defined in Claim 1, so as to obtain the corresponding compound of formula (I).

9. Method for preparing the compounds of formula (I) according to any one of Claims 1 to 7, **characterized in that** a compound of formula (IIa) or (IIb) is reacted with an aryl of formula (III') HO-C₆H₄-CHO, in the presence of a base, the phenylene being unsubstituted or substituted with R⁵, in order to obtain a compound of formula (IVa) or (IVb) 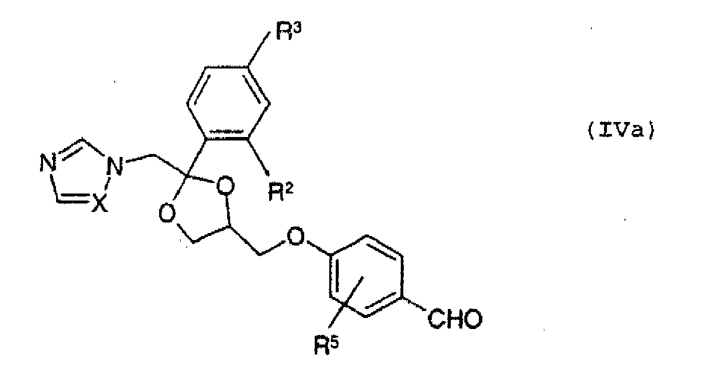 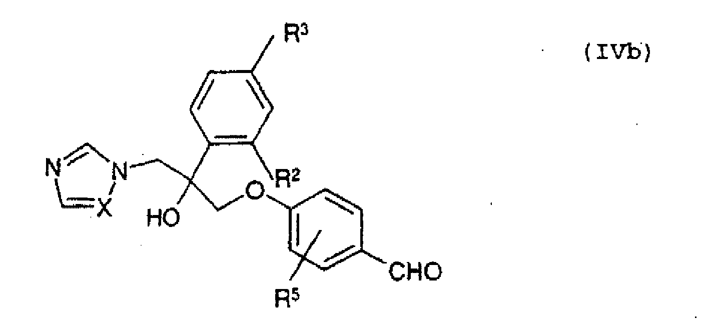 which is reacted in succession
a) with a phosphorane of the (EtO)₂POCH₂CO₂Et type, so as to obtain the corresponding allyl ester,
b) with a reducing agent so as to obtain the corresponding alcohol,
c) with an oxidizing agent so as to form the corresponding aldehyde,
d) with a reducing agent such as NaBH₃CN, in order to obtain the compounds of formula (Ia) or (Ib).

10. Compounds of formula (Ia) or (Ib) as defined in any one of Claims 1 to 7, and also their pharmaceutically acceptable addition salts and/or their prodrugs, in the form of esters of carboxylic, sulphonic or phosphonic groups, or in acyl and carbonate form for the groups containing an acid that can be acylated, or in the form of quaternary derivatives of cyclic or noncyclic nitrogen, as a medicinal product.

11. Compounds of formula (Ia) or (Ib) as defined in any one of Claims 1 to 7, and also their pharmaceutically acceptable addition salts and/or their prodrugs, in the form of esters of carboxylic, sulphonic or phosphonic groups, or in acyl and carbonate form for the groups containing an acid that can be acylated, or in the form of quaternary derivatives of cyclic or noncyclic nitrogen, for their use as an antifungal agent.

12. Application of chemical entities having, at one end, the following groups: 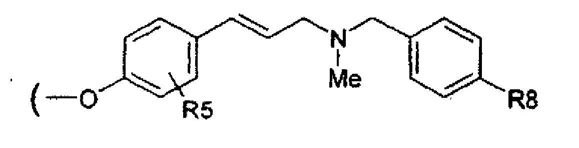 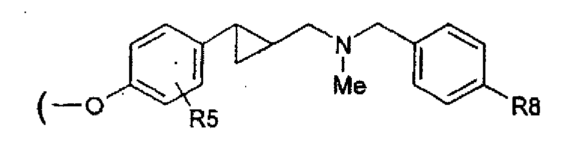 and, at the other end, a pharmacophore group having a fungicidal activity, as defined in Claims 1 and 2, for the preparation of medicinal products having antifungal activity.

13. Pharmaceutical composition containing at least one compound of formula (Ia) or (Ib) as defined in any one of Claims 1 to 7, and also their pharmaceutically acceptable addition salts and/or their prodrugs, in the form of esters of carboxylic, sulphonic or phosphonic groups, or in acyl and carbonate form for the groups containing an acid that can be acylated, or in the form of quaternary derivatives of cyclic or noncyclic nitrogen, and a pharmaceutically acceptable vehicle.

## Patentansprüche

1. Verbindungen der Formel (Ia) und (Ib) 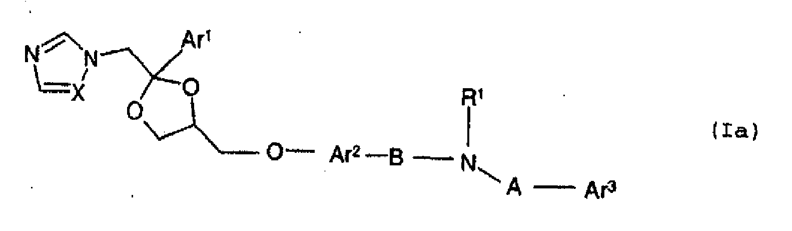 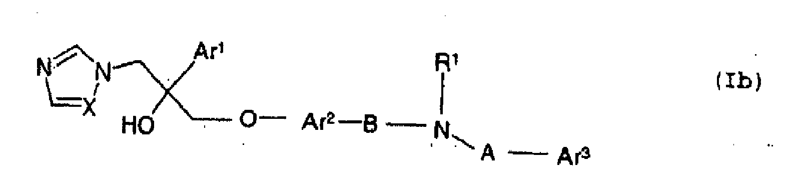 worin
- X für ein Stickstoffatom oder eine CH-Gruppe steht,
- Ar¹ für ein carbocyclisches oder heterocyclisches Aryl, das gegebenenfalls durch einen oder mehrere Reste R², R³ oder R⁴ substituiert ist, steht,
- Ar² für ein Phenylen oder Naphthylen, das gegebenenfalls durch einen oder mehrere Reste R⁵, R⁶ oder R⁷ substituiert ist, steht,
- Ar³ für ein carbocyclisches oder heterocyclisches Aryl, das gegebenenfalls durch einen oder mehrere Reste R⁸, R⁹ oder R¹⁰ substituiert ist, steht,
- A für einen (C₁-C₄)-Alkylenrest steht,
- B für einen -CH=CH-(C₁-C₉)-Alkylenrest oder einen -Cyclopropylen-(C₁-C₄)-alkylenrest steht, wobei der Cyclopropyl- bzw. -CH=CH-Rest gegebenenfalls durch einen Rest R² und/oder R³ substituiert ist,
- R¹ für ein Wasserstoffatom, eine -SO₃H-Gruppe oder auch einen (C₁-C₆)-Alkylrest, der gegebenenfalls durch einen wie für R² definierten Rest substituiert ist, oder einen an die Gruppe Ar³ gebundenen Alkylenrest steht,
- R², R³, R⁴, R⁵, R⁶, R⁷, R⁸, R⁹ oder R¹⁰ gleich oder verschieden sind und für Fluor, Chlor, Brom, Cyano, Mono-, Di- oder Trihalogen-(C₁-C₈)-alkyl, Mono-, Di- oder Trihalogen-(C₁-C₈)-alkoxy, Hydroxy, Nitro, Carboxyl, Formyl, -SO₃H, -OSO₃H, (R¹¹O)₂P(O)-, (R¹¹O)₂P(O)-O-, Amino, (C₁-C₈)-Alkylamino, Di((C₁-C₈)-alkyl)amino, (C₅-C₁₄)-Aryl-(C₁-C₆) -alkylenamino oder (C₅-C₁₄)-Arylamino, (C₁-C₈-Alkyl, (C₅-C₁₄)-Aryl, einen Heterocyclus, der gegebenenfalls durch Oxo substituiert ist, (C₅-C₁₄)-Aryl-(C₁-C₆)-alkyl, Amino-(C₁-C₆)-Alkyl, (C₁-C₈)-Alkylamino-(C₁-C₆)-alkyl, Di((C₁-C₈)-alkyl)amino-(C₁-C₆)-alkyl, Hydroxy-(C₁-C₆)-alkyl, (C₁-C₆)-Alkyloxy-(C₁-C₆)-alkyl, (C₁-Cₐ)-Alkyloxy, das gegebenenfalls durch ein oder mehrere Sauerstoffatome unterbrochen ist, (C₅-C₁₄)-Aryl-(C₁-C₆)-alkylenoxy, (C₅-C₁₄) -Aryloxy, Hydroxy- (C₁-C₆)-alkylenoxy, (C₁-C₆) -Alkyloxy- (C₁-C₆) -alkylenoxy, Amino- (C₁-C₆) -alkylenoxy, (C₁-C₆) -Alkylamino- (C₁-C₆)-alkylenoxy, Di((C₁-C₈)-alkyl) amino- (C₁-C₆)-alkylenoxy, Methylendioxy, (C₁-C₆)-Alkyloxycarbonyl, (C₁-C₆)-Alkylcarbonyl, (C₅-C₁₄) -Aryl- (C₁-C₆) -alkylencarbonyl, (C₅-C₁₄)-Arylcarbonyl, (C₁-C₆)-Alkylaminocarbonyl, (C₁-C₆)-Alkanoylamino, (C₁-C₆)-Alkylsulfonylamino, (C₅-C₁₄)-Arylsulfonylamino, (C₅-C₁₄)-Aryl-(C₁-C₆)-alkylensulfonylamino, (C₁-C₆)-Alkylaminosulfonyl, (C₅-C₁₄)-Aryl-(C₁-C₆)-alkylenaminosulfonyl, (C₁-C₆)-Alkylsulfonyl, (C₅-C₁₄)-Aryl-(C₁-C₈)-alkylensulfonyl oder (C₅-C₁₄)-Arylsulfonyl stehen, wobei die Alkyl-, Aryl- oder Heterocyclusreste selbst gegebenenfalls durch eine oder mehrere der oben angegebenen Gruppen substituiert sein können,
- R¹¹ für Wasserstoff, (C₁-C₁₀)-Alkyl, (C₆-C₁₄)-Aryl oder (C₆-C₁₄)-Aryl-(C₁-C₆)-aryl steht,
in allen ihren möglichen stereoisomeren Formen und Gemische davon sowie physiologisch annehmbare Additionssalze davon und Prodrugs davon in Form von Estern von Carbonsäure-, Sulfonsäure- oder Phosphonsäuregruppen oder in Acyl- und Carbonatform für die Gruppen, die eine acylierbare Säure enthalten, oder in Form von quartären Derivaten von cyclischem oder nichtcyclischem Stickstoff.

2. Verbindungen der Formel (Ia) oder (Ib) nach Anspruch 1, worin A für eine -CH₂-Gruppe steht, B für eine -CH=CH-CH₂- oder -Cyclopropyl-CH₂-Gruppe steht und Ar¹ für ein Phenyl steht und Ar² für ein Phenylen steht, sowie die physiologisch annehmbaren Additionssalze davon.

3. Verbindungen der Formel (Ia) oder (Ib) nach Anspruch 1 mit der Struktur: 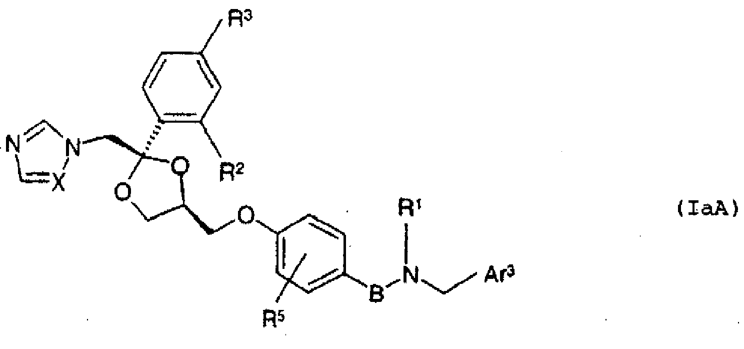 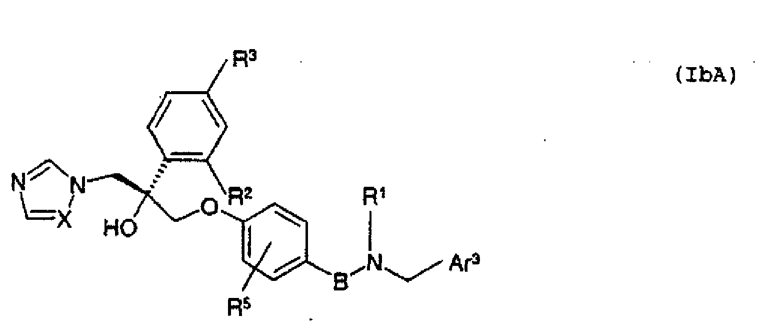 worin B, X, Ar³, R⁵ und R¹ die in Anspruch 1 angegebene Bedeutung besitzen und R² und R³ für ein Halogenatom stehen, sowie die physiologisch annehmbaren Additionssalze davon.

4. Verbindungen der Formel (Ia), (Ib), (IaA) oder (IbA) nach einem der Ansprüche 1 bis 3, worin R² und R³ für Chloratome stehen, X für CH oder N steht und Ar³ für eine Phenylgruppe, die gegebenenfalls durch R⁶ gemäß Anspruch 1 substituiert ist, steht, sowie die physiologisch annehmbaren Additionssalze davon.

5. Verbindungen der Formel (Ia), (Ib), (IaA) oder (IbA) nach einem der Ansprüche 1 bis 4, worin R¹ für ein Wasserstoffatom oder eine Methyl- oder Ethylgruppe, die gegebenenfalls durch eine F-, OH-, NH₂-, (C₁-C₆)-Alkyloxy-, (C₁-C₆)-Alkylamino-, Di (C₁-C₆)-alkylamino-, Pyrrolidino- oder 2-Oxopyrrolidinogruppe substituiert ist, steht, sowie die physiologisch annehmbaren Additionssalze davon.

6. Verbindungen der Formel (Ia), (Ib), (IaA) oder (IbA) nach einem der Ansprüche 1 bis 5, worin Ar³ für Phenyl, das gegebenenfalls durch R⁸, das für einen -Cl-, -F-, CN-, -CF₃-, -OCF₃-, -OH-, -NH₂-, (C₁-C₆)-Alkyloxy-, (C₁-C₆)-Alkylamina- oder Di(C₁-C₆)-alkylaminorest steht, substituiert ist, oder einen unter: 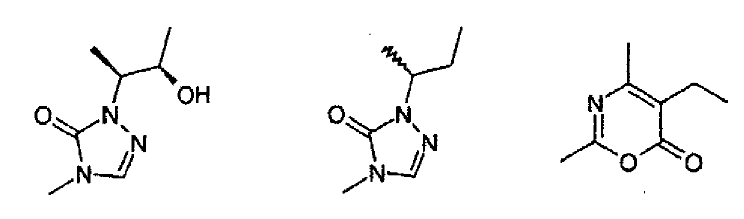 ausgewählten Heterocyclus steht, sowie die physiologisch annehmbaren Additionssalze davon.

7. Verbindungen der Formel (Ib) nach Anspruch 1 mit den folgenden Namen:
- alpha-(2,4-Dichlorphenyl)-alpha-[[4-[3-[[methyl(phenylmethyl)]amino]]-1(E)-propenyl]-phenoxy]methyl]-1H-imidazol-1-ethanol,
- alpha-(2,4-Dichlorphenyl)-alpha-[[4-[3-[[methyl(1-naphthalinylmethyl)]amino]]-1(E)-propenyl]phenoxy]methyl]-1H-imidazol-1-ethanol.

8. Verfahren zur Herstellung von Verbindungen der Formel (Ia) oder (Ib) nach einem der Ansprüche 1 bis 7, **dadurch gekennzeichnet, daß** man eine Verbindung der Formel (IIa) oder (IIb) 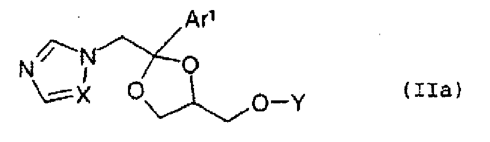 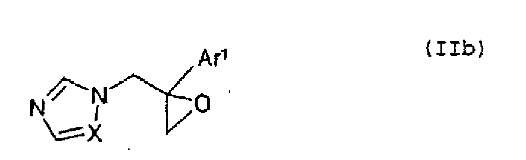 worin Y für eine Abgangsgruppe nach nucleophiler Substitution wie Mesylat oder Tosylat steht und die anderen Substituenten ihre vorhergehende Bedeutung behalten, in Gegenwart einer Base mit einer Verbindung der Formel (III):
HO-Ar²-B-N(R¹)-A-Ar³ (III)
worin Ar², A, R¹, B und Ar³ die in Anspruch 1 angegebene Bedeutung besitzen, zu der entsprechenden Verbindung der Formel (I) umsetzt.

9. Verfahren zur Herstellung von Verbindungen der Formel (I) nach einem der Ansprüche 1 bis 7, **dadurch gekennzeichnet, daß** man eine Verbindung der Formel (IIa) oder (IIb) in Gegenwart einer Base mit einer Arylverbindung der Formel (III') HO-C₆H₄-CHO, wobei das Phenylen gegebenenfalls durch R⁵ substituiert ist, zu einer Verbindung der Formel (IVa) bzw. (IVb): 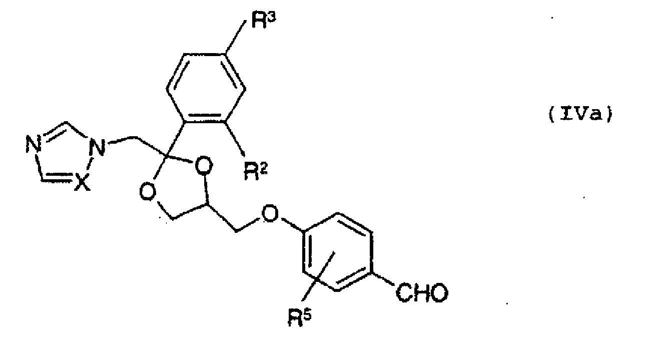 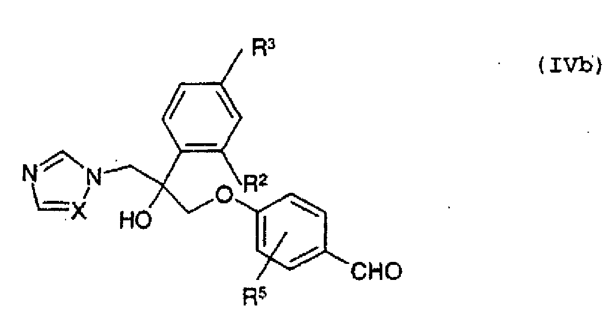 umsetzt, die man dann nacheinander
a) mit einem Phosphoran des Typs (EtO)₂POCH₂CO₂Et zu dem entsprechenden Allylester,
b) mit einem Reduktionsmittel zu dem entsprechenden Alkohol,
c) mit einem Oxidationsmittel zu dem entsprechenden Aldehyd,
d) mit einem Reduktionsmittel wie NaBH₃CN zu den Verbindungen der Formel (Ia) bzw. (Ib)
umsetzt.

10. Verbindungen der Formel (Ia) oder (Ib) nach einem der Ansprüche 1 bis 7 sowie die pharmazeutisch annehmbaren Additionssalze davon und/oder Prodrugs davon in Form von Estern von Carbonsäure-, Sulfonsäure- oder Phosphonsäuregruppen oder in Acyl- und Carbonatform für die Gruppen, die eine acylierbare Säure enthalten, oder in Form von quartären Derivaten von cyclischem oder nichtcyclischem Stickst off als Medikament.

11. Verbindungen der Formel (Ia) oder (Ib) nach einem der Ansprüche 1 bis 7 sowie die pharmazeutisch annehmbaren Additionssalze davon und/oder Prodrugs davon in Form von Estern von Carbonsäure-, Sulfonsäure- oder Phosphonsäuregruppen oder in Acyl- und Carbonatform für die Gruppen, die eine acylierbare Säure enthalten, oder in Form von quartären Derivaten von cyclischem oder nichtcyclischem Stickstoff zur Verwendung als Antimykotikum.

12. Verwendung von chemischen Individuen, die an einem Ende die folgenden Gruppen: 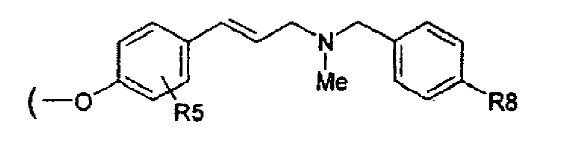 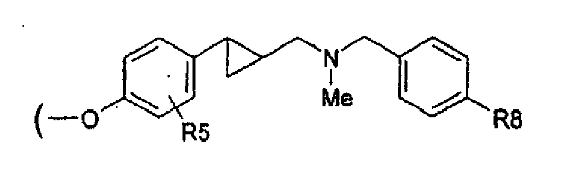 und am anderen Ende eine Pharmakophorgruppe mit fungizider Wirkung nach Anspruch 1 und 2 aufweist, zur Herstellung von Medikamenten mit antimykotischer Wirkung.

13. Pharmazeutische Zusammensetzung, enthaltend mindestens 1 Verbindung der Formel (Ia) oder (Ib) nach einem der Ansprüche 1 bis 7 sowie die pharmazeutisch annehmbaren Additionssalze davon und/oder Prodrugs davon in Form von Estern von Carbonsäure-, Sulfonsäure- oder Phosphonsäuregruppen oder in Acyl- und Carbonatform für die Gruppen, die eine acylierbare Säure enthalten, oder in Form von quartären Derivaten von cyclischem oder nichtcyclischem Stickstoff und einen pharmazeutisch annehmbaren Träger.
